(19) Europäisches Patentamt
European Patent Office
Office européen des brevets

(11) EP 4 197 536 A1

(12) # EUROPEAN PATENT APPLICATION

(43) Date of publication:
**21.06.2023 Bulletin 2023/25**

(21) Application number: **21383162.1**

(22) Date of filing: **20.12.2021**

(51) International Patent Classification (IPC):
***A61K 31/4515*** *(2006.01)* ***A61K 31/4709*** *(2006.01)*
***A61K 31/519*** *(2006.01)* ***A61K 31/7125*** *(2006.01)*
***A61P 21/00*** *(2006.01)* ***A61K 31/711*** *(2006.01)*

(52) Cooperative Patent Classification (CPC):
(C-Sets available)
**A61P 21/00; A61K 31/4515; A61K 31/4709;**
**A61K 31/519; A61K 31/711; A61K 31/7125** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Universitat de València**
  **46010 Valencia (ES)**
- **CECS**
  **91100 Corbeil Essonnes (FR)**
- **INSERM - Institut National de la Santé et de la Recherche Médicale**
  **75013 Paris (FR)**
- **Universite d'Evry Val d'Essonne**
  **91025 Evry (FR)**
- **Università Degli Studi Di Torino**
  **10124 Torino (IT)**

(72) Inventors:
- **Konieczny, Piotr Tadeusz**
  **46035 Valencia (ES)**
- **Artero Allepuz, Rubén Darío**
  **46130 Valencia (ES)**
- **Januel, Camille**
  **75020 París (FR)**
- **Martinat, Cécile**
  **91600 Savigny sur Orge (FR)**
- **Boido, Marina Maria**
  **10042 Nichelino (Turín) (IT)**
- **Menduti, Giovanna**
  **84091 Battipaglia (Salerno) (IT)**

(74) Representative: **Manuel Illescas y Asociados, S.L.**
**C/ Príncipe de Vergara 197, Oficina 1°A**
**28002 Madrid (ES)**

Remarks:
The complete document including Reference Table(s) and the Sequence Listing(s) can be downloaded from the EPO website

(54) **HALOPERIDOL FOR USE IN THE TREATMENT OF SPINAL MUSCULAR ATROPHY**

(57) Haloperidol, or a pharmaceutically acceptable salt, ester or solvate thereof, as well as pharmaceutical compositions comprising the same, for use in methods for preventing and/or treating spinal muscular atrophy, are described.

EP 4 197 536 A1

(52) Cooperative Patent Classification (CPC): (Cont.)

C-Sets
**A61K 31/4515, A61K 2300/00;**
**A61K 31/4709, A61K 2300/00;**
**A61K 31/519, A61K 2300/00;**
**A61K 31/711, A61K 2300/00;**
**A61K 31/7125, A61K 2300/00**

## Description

## FIELD OF THE INVENTION

The present invention belongs to the pharmaceutical sector and refers to haloperidol or a pharmaceutically acceptable salt or solvate thereof, as well as pharmaceutical compositions comprising the same, for use in methods for preventing and/or treating spinal muscular atrophy.

## BACKGROUND OF THE INVENTION

Spinal muscular atrophy (SMA) is a rare genetic neuromuscular disorder caused by loss of alpha-motor neurons in spinal cord and brain nuclei. The muscle atrophy in SMA was believed to be solely a consequence of muscle fiber denervation; however, there is more and more evidence that muscle defects take place independently and even prior to motor neuron degeneration. The experiments in SMA murine model and human cell revealed defects in the myoblast's proliferation, maturation and fusion processes. Every 1 in 6000 to 10 000 children is affected by the disease and every 1 in 40 people is a carrier of the responsible mutation, which shows a recessive autosomal inheritance pattern.

SMA is primarily caused by mutations in the SMN1 gene. However, as a result of duplication in the genome, the SMN locus (chromosome location 5q13) contains two inverted copies of SMN, called SMN1 (telomeric) and SMN2 (centromeric). The SMN1 and SMN2 genes are almost identical with only 5 nucleotide changes: one in intron 6 (G/A), exon 7 (C/T), exon 8 (G/A) and two in intron 7 (AA/GG). The difference in the coding regions of both genes is a C-to-T transition at position +6 of exon 7 in SMN2. This change is critical, as it results in a different splicing pattern. In SMN1, exon 7 (Ex7) is included, resulting in a full-length and functional SMN protein. However, the C-to-T transition in SMN2 causes Ex7 exclusion in most cases, resulting in a C-terminally truncated SMN protein (Δ7-SMN). Nevertheless, a fraction of the SMN2 mRNAs include Ex7 and results in a functional, full-length SMN protein (FL-SMN), which is, however, insufficient to compensate for the SMN1 loss. Additionally, the truncated SMN protein comprising 282 instead of 294 amino acids has an impaired oligomerization capability and is highly unstable.

All the patients suffering SMA carry one or more copies of SMN2 gene, which is mostly translated into the unfunctional truncated SMN isoform, and producing only a small part of functional protein.

Despite even the most severe cases of SMA normally manifest at postnatal stages, there are studies that report increased motor neuron loss, decreased myotube size and abnormal synaptic contacts at neuromuscular junctions (NMJ), at the prenatal stages. In fact, even though motor neurons are the main cell type affected in the SMN physiopathology, there is increasing evidence of the multisystemic character of SMA, affecting directly muscles, heart, brain and pancreas.

The discovery of the mutated SMN1 gene and its unaffected SMN2 copy, allowed linking the SMN2 copy number with the disease severity (see table 1). In fact, the number of SMN2 copies is inversely correlated with the disease severity because the amount of functional protein translated from the SMN2 transcript, partially compensate for SMN deficiencies caused by the mutations in SMN1.

**Table 1:** SMA type

| SMA type | Age of Onset | Lifespan | SMN2 copy number |
|---|---|---|---|
| IA | < 1 week/congenital | < 1 month | 1 |
| IB | 1 week - 3 months | < 2 years | 2,3 |
| IC | 3 - 6 months | < 2 years | 2,3 |
| IIA | 6 - 15 months | > 2 years | 2,3,4 |
| IIB | 6 - 15 months | > 2 years | 2,3,4 |
| IIIA | < 3 years | Adult | 3,4 |
| IIIB | > 3 years | Adult | 3,4 |
| IV | > 21 years | Adult | 4, 5 |

On the other hand, despite the fact that the presence of the SMN2 copy does not fully compensate for the SMN1 loss of function, it constitutes an important therapeutic target in many aspects. In fact, the specific enhancement of SMN2 Ex7 inclusion is a promising therapeutic approach, which increases the full-length transcript levels and, as a consequence, the functional SMN protein levels. The full-length to truncated protein ratio in patients is approximately

1:4, and accordingly, a therapy increasing the Ex7 inclusion will be an efficient therapeutic approach.

Two approaches are currently being explored: one involves the use of Ex7 inclusion promoting antisense oligonucleotides (ASOs), and the other one is based on small molecules with similar splicing switching properties.

ASOs are single-stranded oligonucleotides designed to be complementary to a targeted RNA sequence. The ASOs can be designed for modulation of splicing or RNA processing. This strategy was proposed for designing therapeutic oligonucleotides that modify the SMN2 pre-mRNA alternative splicing, favoring the Ex7 inclusion in the final transcript. For instance, recently, a 2'-O-methoxyethyl-ASO-based therapy called nusinersen (Spinraza™) has become the first FDA-approved therapy for the severe SMA, although additional clinical trials are still being carried out. ASOs do not cross the hematoencephalic barrier when administered in a systemic route and, accordingly, nusinersen requires intrathecal administration. The nusinersen administration protocol includes two phases: one initial charge doses, with a frequency of four injections during two months and, subsequently, a maintenance phase wherein injections are administered every four months.

Another SMA therapy recently approved (2020) for adults and children older than 2 months, is risdiplam (Evrysdi®). Risdiplam interacts directly with the spliceosome to promote exon 7 inclusion in the pre-mRNA of *SMN2* producing an increase in the level of the full length functional SMN protein. It is a liquid drug administered orally, since it is capable to cross the hematoencephalic barrier. This allows treatment without hospitalization and a systemic distribution of the drug, not only through the central nervous system but also in other organs and peripheric tissues.

Using the same therapeutic target, i.e. promoting the expression of the SMN protein, Zolgensma™ (Onasemnogene abeparvovec) was approved in 2019 for pediatric SMA patients under the age of 2 years. Onasemnogene abeparvovec is a gene therapy for the replacement of SMN1, independently of gene SMN2, i.e., it is directed directly to the dysfunctional gene causing SMA. This replacement is achieved using a non-replicating adeno-associated virus capsid (scAAV9). The administration of this therapy consists in a single intravenous systemic application, since it crosses the hematoencephalic barrier and also enters in motor neurons, muscle and other tissues where SMN is highly expressed, resulting in a systemic and durable increase of SMN1 expression as well as a longer survival of the patient, since the protein is ubiquitously expressed.

Despite the existence of several approved therapies, there is still the need for therapeutic alternatives to overcome the disadvantages and limitations of the current approved drugs. In fact, the currently marketed therapies include several limitations or disadvantages: invasive administrations routes (nusinersen), efficacy dependent of patient age and type (more effective when patients are treated at earlier stages), adverse effects and high cost.

For instance, nusinersen administration route is highly invasive, since it is administered intrathecally, several times per year, during the whole life of the patient's life and, accordingly, requires hospitalization periods. Moreover, this administration route may include several difficulties for patients with serious scoliosis or having suffered a spinal fusion. Analogously, the presence of low levels of the SMN protein in peripheric tissues, due to the lack of systemic availability of the drug due to the administration route and the consequence thereof is also a concern when using nusinersen.

The development of drugs that are systemically administered, such as onasemnogene abeparvovec, or with oral administration, such as risdiplam, has overcome some of the issues raised by nusinersen administration route. However, onasemnogene abeparvovec has been only approved for patients of age 2 or younger, and the presence of preexistent anti-AAV9 antibodies in the SMA patient population, or the presence of antibodies developed after the treatment may also be an issue, since it may reduce the therapeutic benefits of this therapy and increase the immune response risks.

Additionally, the occurrence of important adverse effects may appear in patients being administered those treatments. Among those adverse effects are headaches and back pain due to nusinersen administration, acute hepatic damage, bleeding and cardiac damage due to onasemnogene abeparvovec. Additionally, since risdiplam has not been used for long periods, it is still unknown the possible adverse effects that may cause long term, as well as the risks that may pose in other areas. Moreover, risdiplam features a low capability to cross the hematoencephalic barrier.

Lastly, one of the most important limitations of all the approved therapies is their high cost, which prevents the access thereto to many patients. For instance, the first treatment year with nusinersen represents currently a cost of about 750,000 USD, and 375,000 USD for the following years of chronic treatment. On the other hand, the price of risdiplam represents about 340,000 USD per year and the single dose, and onasemnogene abeparvovec is the world's most expensive drug costing the required single dose about 2.1 million USD.

Accordingly, there is still a need to develop new treatments for SMA, with a more convenient, more affordable and feature a less invasive administration regime, which are able to provide good levels of the full-length, functional SMN protein and have a good capability to cross the hematoencephalic barrier. In particular, a drug capable of increasing the amount of full-length functional SMN protein in different body tissues, in particular, in motor neurons, with a simple administration route and accessible costs is still needed.

## BRIEF DESCRIPTION OF THE INVENTION

The present invention relates to haloperidol, or a pharmaceutically acceptable salt, ester or solvate thereof, for use in a method for preventing and/or treating spinal muscular atrophy, wherein said method results in an increase of full length functional SMN protein levels.

Another aspect of present invention also refers to haloperidol, or a pharmaceutically acceptable salt, ester or solvate thereof, for use in a method for preventing and/or treating spinal muscular atrophy, wherein said method increases the SMN2 transcript expression and/or increases of SMN2 exon 7 inclusion and, accordingly, results in an increase of full length functional SMN protein levels.

Additionally, present invention refers to a pharmaceutical composition comprising haloperidol, or a pharmaceutically acceptable salt, ester or solvate thereof, and at least a pharmaceutically acceptable excipient; for use in a method for preventing and/or treating spinal muscular atrophy, wherein said method results in an increase functional SMN protein levels.

Yet another aspect of present invention refers to a pharmaceutical composition comprising haloperidol, or a pharmaceutically acceptable salt, ester or solvate thereof, and at least a pharmaceutically acceptable excipient; for use in a method for preventing and/or treating spinal muscular atrophy, wherein said method increases the SMN2 transcript expression and/or increases of SMN2 exon 7 inclusion and, accordingly, results in an increase of full length functional SMN protein levels.

Finally, another aspect of present invention refers to a pharmaceutical composition comprising haloperidol and moxifloxacin, or a pharmaceutically acceptable salt, ester or solvate thereof, and at least a pharmaceutically acceptable excipient, wherein said pharmaceutical composition comprises a molar ratio of haloperidol:moxifloxacin from 1:5 to 1:10.

## BRIEF DESCRIPTION OF THE FIGURES

**Fig. 1. Fusion index of immortalized myoblasts derived from patients with SMA type II. (A)** The cells were differentiated initially for 3 days and subsequently treated with haloperidol, for the following 72 h. Healthy and solvent treated SMA myoblasts (0.5% DMSO) was used to establish a difference in the differentiation process. Risdiplam at 1 μM was used as a positive control. Nuclei were labeled with 4',6-diamidino-2-phenylindole (DAPI) and myoblasts were detected using an anti-Desmin antibody. Multinuclear myotubes are marked with white arrows. The fusion index of DMSO-treated healthy and SMA differentiated myoblasts, and compound-treated SMA myoblasts, was calculated. Data were collected after analysis of at least 100 cells and statistics were calculated using Student's t-test (* $p < 0.05$, ** $p < 0.01$, *** $p < 0.001$). **(B)** Representative images of the experiment were acquired under confocal microscopy.

**Figure 2: Effect of Haloperidol treatment on SMA motoneurons survival rate. (A)** Representative image of motoneurons stained using neural marker (TUJ1) and merge photos for cell nucleus (DAPI), MNs differentiation marker (ISL1) and SMN protein (SMN) after treatment with haloperidol at 320 nM, compared to healthy MNs and DMSO solvent control. **(B)** MNs survival rate analysis after 10 days treatment at range of concentrations. **(C)** Relative SMN expression levels (delta F) measured by a Homogeneous Time Resolved Fluorescence (HTRF) Kit.

**Figure 3: Effect of Haloperidol treatment on motoneurons (MNs) and myoblasts co-culture. (A)** MNs survival rate analysis after haloperidol treatment compared to non-treated controls. **(B)** Analysis of SMA myotubes area compared to the non-treated controls. **(C)** Representative images of co-culture treated with haloperidol at 320 nM, compared to solvent-treated wild-type and SMA cells. Myotubes were marked using MF20 marker (dark green), MNs were marked with ISL1 (red), acetylcholine receptors were marked with AChR (light green) and cell nuclei were stained with Hoechst (blue).

**Fig. 4. Effect of Haloperidol treatment on weight and motor performances of SMN Δ7 mice.** Starting from P5 (postnatal day 5), treated mice exhibit remarkable weight gain **(A)** and significant motor performance improvements, as demonstrated by tail suspension **(B),** righting reflex **(C)** and negative geotaxis tests **(D)** VHL (vehicle) n=8, HALO (haloperidol) n=5. Results are expressed as mean ± SEM (two-way ANOVA, * $p \leq 0.05$; ** $p \leq 0.01$; *** $p \leq 0.001$; **** $p \leq 0.0001$).

**Fig. 5. Analysis of SMN expression by Western Blot.** At P12, the SMN expression was evaluated in both spinal cord **(A1** and **A2),** diaphragm **(B1** and **B2),** quadriceps **(C1** and **C2)** and gastrocnemius **(D1** and **D2)** samples: we respectively observed a 58%, 23%, 123% and 102% SMN expression increase in HALO (haloperidol) treated mice

compared to VHL (vehicle) treated mice (3 HALO vs 3 VHL).

**Fig. 6. Effect of haloperidol and moxifloxacin on SMA motoneurons survival rate.** Cells were treated with 7 concentrations of each drug separately and the survival rate was measured after 10 days of treatment.

**Figure 7. Effect of haloperidol in combination with moxifloxacin on SMA motoneurons survival rate.** Cells were treated with 49 combinations of haloperidol and moxifloxacin at different concentrations and the survival rate was measured after 10 days of treatment.

**Figure 8. Bidimensional representation of the analysis of the synergistic effect.** The synergistic and antagonistic effects of haloperidol and moxifloxacin combinations was plotted on a two-dimensional contour plot where the x-axis and y-axis represent doses of haloperidol and moxifloxacin, respectively.

## DETAILED DESCRIPTION

The present invention refers to haloperidol, or a pharmaceutically acceptable salt, ester or solvate thereof, for use in a method for preventing and/or treating spinal muscular atrophy, wherein said method results in an increase of full length functional SMN protein levels. Haloperidol is an antipsychotic drug, known to cross the hematoencephalic barrier, and is a strong antagonist of the dopamine receptor 2, which is indicated for the treatment of the manifestations of several psychotic disorders including schizophrenia, acute psychosis, Tourette syndrome, and other severe behavioral disorders. Additionally, haloperidol has also been used for the management of chorea associated with Huntington's disease or in palliative care, probably, as an antiemetic agent.

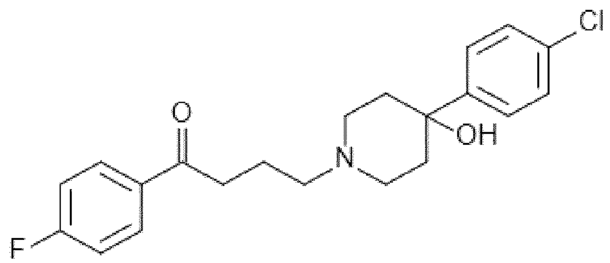

Haloperidol

To that respect, present invention shows that, surprisingly, in addition to the metabolic effects known in the art, haloperidol administration results in an increase of full length functional SMN protein levels, effect that makes said compound useful in the treatment of spinal muscular atrophy.

In fact, Example 1 shows that haloperidol produced a significant increase in SMN protein levels and, in particular, produced a significant increase in the SMN2 functional full length isoform, as shown in Example 2.

Accordingly, present invention refers to haloperidol, or a pharmaceutically acceptable salt, ester or solvate thereof, for use in a method for preventing and/or treating spinal muscular atrophy, wherein said method results in an increase of full length functional SMN protein levels, in particular, wherein said method increases the SMN2 transcript expression and/or increases of SMN2 exon 7 inclusion.

The term "pharmaceutically acceptable salt" refers to any pharmaceutically acceptable salt, which upon administration to the patient is capable of providing (directly or indirectly) a compound as described herein. Such salts preferably are acid addition salts with physiologically acceptable organic or inorganic acids, or alkali addition salts with acceptable organic or inorganic bases. Examples of the acid addition salts include mineral acid addition salts such as, for example, hydrochloride, hydrobromide, hydroiodide, sulphate, nitrate, phosphate, and organic acid addition salts such as, for example, lactate, acetate, trifluoroacetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium and ammonium salts, and organic alkali salts such as, for example, ethylenediamine, ethanolamine, N,N-dialkylenethanolamine, triethanolamine and basic aminoacids salts. However, it will be appreciated that non-pharmaceutically acceptable salts also fall within the scope of the invention since those may be useful in the preparation of pharmaceutically acceptable salts. Procedures for salt formation are conventional in the art. Preferably, said pharmaceutically acceptable haloperidol salt is haloperidol lactate.

The term "pharmaceutically acceptable ester" refers to any form of the active compound in accordance with the invention in which the hydroxyl group of haloperidol forms an ester with a carboxylic acid. Preferably, said pharmaceutically acceptable haloperidol ester is haloperidol decanoate.

The term "pharmaceutically acceptable solvate" in accordance with this invention should be understood as meaning any form of the active compound in accordance with the invention in which said compound is bonded by a non-covalent bond to another molecule (normally a polar solvent), including especially hydrates and alcoholates.

Present invention shows, therefore, that haloperidol, a pharmaceutically acceptable salt, ester or solvate thereof, is useful in the prevention and/or treatment of SMA. In this sense, the examples of present application show that, in addition to the effect of haloperidol in the SMN protein levels, treatment with haloperidol had, as a consequence effects in myoblasts and motor neurons.

In fact, SMN-deficient myoblasts, as those from SMA patients, have been reported to have impaired fusion capabilities. To that respect, myoblasts treated with haloperidol significantly increased the fusion index, recovering this capacity up to 66.7% (compared to a fusion rate of about 68% for healthy myoblasts) when patient myoblasts have reduced it to 50% (Figure 1A). Moreover, haloperidol administration also resulted in an increased survival rate of motor neurons (Figure 2B and 3A) and increased the area of myotubes in respect to non-treated controls (figure 3B).

Finally, in vivo assays carried out in a murine SMA model showed that haloperidol administration resulted in a significant weight increase in treated SMA mice, and, in addition, the behavioral assessment showed that haloperidol administration improved motor performances (Figure 4). In fact, the behavioral results and improved motor performances positively correlated with the increase in SMN expression in several tissues (spinal cord, diaphragm, quadriceps and gastrocnemius) provided by haloperidol administration (Figure 5) and was not associated with any conspicuously deleterious or undesirable side effects, such as behavioral changes or sedation phenotypes.

Accordingly, present invention also refers to a pharmaceutical composition comprising haloperidol, or a pharmaceutically acceptable salt, ester or solvate thereof, and at least a pharmaceutically acceptable excipient; for use in a method for preventing and/or treating spinal muscular atrophy, wherein said method results in an increase of full length functional SMN protein levels, in particular, wherein said method increases the SMN2 transcript expression and/or increases of SM N2 exon 7 inclusion and, accordingly, results in an increase of full length functional SMN protein levels.

For the purposes of present invention, the term "comprises" may be replaced by any of the terms "consists of", or "consists essentially of". Accordingly, "comprises" may refer to a group of features A, B and C, which additionally may include other features, such as E and D, with the condition that said features do not render the claim unworkable, but said term "comprises" also includes the situation in which the group of features "consists of" or "consists essentially" of A, B and C.

The excipient included in the pharmaceutical compositions for use according to present invention, refers, for the purpose of present invention, to an inert ingredient such as, but not limited to, cosolvents, surfactants, oils, humectants, emollients, preservatives, stabilizers and antioxidants.

Another aspect of present invention refers to the use of haloperidol, or a pharmaceutically acceptable salt, ester or solvate thereof, or to the use of a pharmaceutical composition comprising the same, for the manufacturing of a medicament for the prevention and/or treatment of spinal muscular atrophy, wherein said medicament results in an increase of full length functional SMN protein levels and, in particular wherein administering said medicament increases the SMN2 transcript expression and/or increases of SMN2 exon 7 inclusion.

Yet another aspect of present invention refers to a method of preventing and/or treating spinal muscular atrophy, wherein said method comprises administering an effective amount of haloperidol, or a pharmaceutically acceptable salt, ester or solvate thereof, or of a pharmaceutical composition comprising the same, to a subject in need thereof. Preferably, said method results in an increase of full length functional SMN protein levels and, in particular, said method increases the SMN2 transcript expression and/or increases of SMN2 exon 7 inclusion.

In a preferred embodiment of the invention, the subject is a pediatric subject. In another preferred embodiment of the invention, the subject is an adult.

A preferred aspect of the invention refers to haloperidol, or a pharmaceutically acceptable salt, ester or solvate thereof, or to a pharmaceutical composition comprising the same, for use in a method for preventing and/or treating spinal muscular atrophy, wherein said haloperidol, or a pharmaceutically acceptable salt, ester or solvate thereof, or pharmaceutical composition comprising the same is administered to a pediatric subject or to an adult.

For the purposes of present invention, an effective amount of the compounds disclosed herein, when said effective amount is referred to a therapeutic use of said compounds, is that which provides a therapeutic effect in the subject or an objectively identifiable improvement in the health condition of said subject as noted by the clinician or other qualified observer.

In a preferred embodiment, said pharmaceutically acceptable salt, ester or solvate thereof, is haloperidol lactate or haloperidol decanoate.

Preferably, the spinal muscular atrophy is type I, type II, type III or type IV. More preferably, the spinal muscular atrophy is type IA, type IB, type IC, type IIA, type IIB, type IIIA, type IIIB or type IV.

In one embodiment of the present invention, the pharmaceutical compositions disclosed herein may optionally comprise at least a second therapeutic agent having additive or synergistic biological activities. For the purposes of

present invention, the term "therapeutic agent" should be taken as synonyms and mean a chemical entity which exerts therapeutic effects when administered to human or animal beings.

Accordingly, in a preferred embodiment of present invention haloperidol, or said pharmaceutically acceptable salt, ester or solvate thereof, or the pharmaceutical composition comprising the same, is administered prior to, together with, or after administering an additional therapeutic agent for use in the treatment of spinal muscular atrophy.

In an embodiment of present invention said additional therapeutic agent is a SMN gene replacement therapy, a SMN2 splicing modifier, a muscle enhancing agent, a p38 MPAK inhibitor, an antimyostatin, a calcium channel modifier or troponin activation therapy. Preferably, said additional therapeutic agent is selected from the group consisting of: moxifloxacin, nusinersen, onasemnogene abeparvovec (AVXS-101), risdiplam (RO703406 or RG7916), BIIB110, Columbia/NU-p38aMAPK inhibitor, branaplam (LMI070), monoclonal antibody SRK-015, albuterol, splicing modifier RG7800, recombinant protein RO7239361 and reldesemtiv, or a combination thereof.

Moxifloxacin (IUPAC name 1-cyclopropyl-7-[(1S,6S)-2,8-diazabicyclo[4.3.0]nonan-8-yl]-6-fluoro-8-methoxy-4-oxoquinoline-3-carboxylic acid is an antibiotic with a quinolone structural core that is able to increase the full length SMN protein levels by promoting exon 7 inclusion in SMN2 in a concentration dependent manner.

Nusinersen is a 2'-O-methoxyethyl phosphorothioate-modified antisense oligonucleotide that promotes exon 7 inclusion in *SMN2* transcripts by blocking through the steric hindrance of a splicing silencer.

Onasemnogene abeparvovec, known previously as AVXS-101 (Zolgensma®) is a gene replacement therapy that uses a non-replicating adeno-associated virus capsid (scAAV9) to deliver a functional copy of the SMN gene to motor neuron cells.

Risdiplam, also known as RO703406 or RG7916 (Evrysdi®), is a small molecule that promotes exon 7 inclusion in pre-mRNA of SMN2, providing a higher expression of the full-length functional SMN protein.

In a preferred embodiment, said additional therapeutic agent is moxifloxacin, nusinersen or risdiplam, or a combination thereof. More preferably, said additional therapeutic agent is nusinersen or risdiplam, or a combination thereof. Even more preferably, said additional therapeutic agent is moxifloxacin or a pharmaceutically acceptable salt, ester or solvate thereof.

In fact, the combined administration of haloperidol and moxifloxacin provides a surprisingly improved activity, providing a synergic effect, as shown in example 7. In fact, example 7, as well as figures 7 and 8, show the synergic effect of the combination of moxifloxacin and haloperidol (combined administration thereof) on SMA motoneurons survival rate using the Bliss Independence model to analyze interaction between drugs (Thas et al., 2021; Van der Borght et al., 2017).

Figures 7 and 8 show that the combination of both drugs, moxifloxacin and haloperidol, improves motoneurons survival to a higher extent than the drugs administered separately, even when moxifloxacin and haloperidol are administered separately at higher doses.

Thus, even more preferably, haloperidol, or said pharmaceutically acceptable salt, ester or solvate thereof, or the pharmaceutical composition comprising the same, is administered prior to, together with, or after administering moxifloxacin, providing said combined administration a synergic (higher than additive) activity, in the prevention and/or treatment of spinal muscular atrophy, when compared to the activity that said compounds provide when administered separately. In a preferred embodiment the haloperidol for use, or a pharmaceutically acceptable salt, ester or solvate thereof is administered together with moxifloxacin, or a pharmaceutically acceptable salt, ester or solvate thereof, preferably in a pharmaceutical composition comprising haloperidol and moxifloxacin, or a pharmaceutically acceptable salt, ester or solvate thereof, and at least one pharmaceutically acceptable excipient.

More preferably said haloperidol for use is administered prior to, together with, or after administering moxifloxacin at a molar ratio of haloperidol:moxifloxacin from 1:5 to 1:10; or said haloperidol for use is administered in a pharmaceutical composition comprising haloperidol and moxifloxacin, or a pharmaceutically acceptable salt, ester or solvate thereof, at a molar ratio of haloperidol:moxifloxacin from 1:5 to 1:10.

Accordingly, another aspect of present invention refers to a pharmaceutical composition comprising haloperidol and moxifloxacin, or a pharmaceutically acceptable salt, ester or solvate thereof, and at least a pharmaceutically acceptable excipient, wherein said pharmaceutical composition comprises a molar ratio of haloperidol:moxifloxacin from 1:5 to 1:10.

One embodiment refers to said pharmaceutical composition comprising haloperidol and moxifloxacin, or a pharmaceutically acceptable salt, ester or solvate thereof, and at least a pharmaceutically acceptable excipient, wherein said pharmaceutical composition comprises a molar ratio of haloperidol:moxifloxacin from 1:5 to 1:10; for use as a medicament, and in particular, for use in the prevention and/or treatment of spinal muscular atrophy.

Also disclosed is the use of said pharmaceutical composition comprising haloperidol and moxifloxacin, or a pharmaceutically acceptable salt, ester or solvate thereof, and at least a pharmaceutically acceptable excipient, in the manufacturing of a medicament for the prevention and/or treatment of spinal muscular atrophy, and wherein said medicament comprises a molar ratio of haloperidol:moxifloxacin from 1:5 to 1:10.

Further disclosed is a method of preventing and/or treating spinal muscular atrophy wherein said method

comprises administering an effective amount of a pharmaceutical composition comprising haloperidol and moxifloxacin, or a pharmaceutically acceptable salt, ester or solvate thereof, and at least a pharmaceutically acceptable excipient, to a subject in need thereof, wherein said pharmaceutical composition comprises a molar ratio of haloperidol:moxifloxacin from 1:5 to 1:10.

Additionally, the combined administration of haloperidol with risdiplam and/or nusinersen provides also a surprisingly improved activity. Accordingly, more preferably, haloperidol, or said pharmaceutically acceptable salt, ester or solvate thereof, or the pharmaceutical composition comprising the same, is administered prior to, together with, or after administering risdiplam and/or nusinersen.

Additionally, the dosage of the compounds and compositions disclosed in accordance with the invention varies depending on the compound and the condition being treated for example the age, weight, and clinical condition of the recipient patient, as well as their use in combination with other therapeutic agents. Other factors include: the route of administration, the patient, the patient's medical history, the severity of the disease process, and the potency of the particular compound. The dose, or effective amount, should be sufficient to ameliorate symptoms or signs of the disease treated without producing unacceptable toxicity to the patient.

One preferred embodiment disclosed herein refers to the route of administration, that may be any route which effectively transports the compounds and compositions disclosed in present disclosure, to the appropriate or desired site of action, such as oral, nasal, topical, pulmonary, transdermal, intrathecal or parenteral, e. g., rectal, subcutaneous, intravenous, intraurethral, intramuscular, intranasal, ophthalmic solution or an ointment.

Preferably, haloperidol, or said pharmaceutically acceptable salt, ester or solvate thereof, or the pharmaceutical compositions comprising haloperidol alone or in combination with other therapeutic agents, and in particular comprising haloperidol and moxifloxacin, is administered orally, intrathecally or parenterally, more preferably orally, intrathecally or via transdermal administration route.

For oral administration, either solid or fluid unit dosage forms can be prepared. For preparing solid compositions such as tablets, the compounds for use according to present invention, are mixed into formulations with conventional ingredients such as talc, magnesium stearate, dicalcium phosphate, magnesium aluminum silicate, calcium sulphate, starch, lactose, acacia, methylcellulose, and functionally similar materials as pharmaceutical diluents or carriers. Capsules are prepared by mixing the compounds for use according to present invention with an inert pharmaceutical diluent and filling the mixture into a hard gelatin capsule of appropriate size. Soft gelatin capsules are prepared by machine encapsulation of slurry of the compound with an acceptable vegetable oil, light liquid petrolatum or other inert oil. Fluid unit dosage forms for oral administration such as syrups, elixirs and suspensions can be prepared. The water-soluble forms can be dissolved in an aqueous vehicle together with sugar, aromatic flavoring agents and preservatives to form syrup. An elixir is prepared by using a hydroalcoholic (e. g., ethanol) vehicle with suitable sweeteners such as sugar and saccharin, together with an aromatic flavoring agent. Suspensions can be prepared with an aqueous vehicle with the aid of a suspending agent such as acacia, tragacanthin, methylcellulose and the like.

The pharmaceutical compositions include the compounds described in present application associated with pharmaceutically acceptable excipients, which may be a carrier or a diluent, as a way of example. Such compositions can be included in a capsule, sachet, paper or other container. In making the compositions, conventional techniques for the preparation of pharmaceutical compositions may be used. For example, the compounds for use according to present invention may be mixed with a carrier, or diluted by a carrier, or enclosed within a carrier that may be in the form of an ampoule, capsule, sachet, paper, or other container. When the carrier serves as a diluent, it may be solid, semi-solid, or liquid material that acts as a vehicle, excipient, or medium for the active compound. The compounds for use according to present invention can be adsorbed on a granular solid container for example in a sachet. Some examples of suitable carriers are water, salt solutions, alcohols, polyethylene glycols, polyhydroxyethoxylated castor oil, peanut oil, olive oil, lactose, terra alba, sucrose, cyclodextrin, amylose, magnesium stearate, talc, gelatin, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose, and polyvinylpyrrolidone. Similarly, the carrier or diluent may include any sustained release material known in the art, such as glyceryl monostearate or glyceryl distearate, alone or mixed with a wax. Said compositions may also include wetting agents, emulsifying and suspending agents, preserving agents, sweetening agents or flavoring agents. The compositions for use according to the invention may be formulated so as to provide quick, sustained, or delayed release of the compounds for use according to present invention after administration to the patient by employing procedures well known in the art.

The pharmaceutical compositions can be sterilized and mixed, if desired, with auxiliary agents, emulsifiers, salt for influencing osmotic pressure, buffers and/or coloring substances and the like, which do not deleteriously react with the compounds for use according to present invention.

In addition to the compounds for use according to present invention, the compositions for use according to the invention may include, depending on the composition and mode of delivery desired, pharmaceutically- acceptable, non-toxic carriers or diluents, which include vehicles commonly used to form pharmaceutical compositions for animal or human administration. The diluents are selected so as not to unduly affect the biological activity of the combination.

Examples of such diluents that are especially useful for injectable formulations are water, the various saline, organic or inorganic salt solutions, Ringer's solution, dextrose solution, and Hank's solution. In addition, the pharmaceutical composition or formulation may include additives such as other carriers; adjuvants; or non-toxic, non-therapeutic, non-immunogenic stabilizers and the like.

Furthermore, excipients can be included in the compositions disclosed. Examples include, but are not limited to, cosolvents, surfactants, oils, humectants, emollients, preservatives, stabilizers and antioxidants. Any pharmacologically acceptable buffer may be used, such as, tris or phosphate buffers. Effective amounts of diluents, additives, and excipients are those amounts that are effective to obtain a pharmaceutically acceptable formulation in terms of solubility, biological activity, etc.

The pharmaceutical compositions comprising the compounds for use according to present invention may be incorporated into a microsphere. The compounds for use according to present invention can be loaded into albumin microspheres, from which it is possible to recover such microspheres in a dry powder for nasal administration. Other materials suitable for the preparation of microspheres include agar, alginate, chitosan, starch, hydroxyethyl starch, albumin, agarose, dextran, hyaluronic acid, gelatin, collagen, and casein. The microspheres can be produced by various processes known to the person skilled in the art such as a spray drying process or an emulsification process.

Another preferred embodiment of the invention is the dosage scheme of the compounds for use according to present invention. The term "unit dosage form" refers to physically discrete units suitable as unitary dosages for subjects, e. g., mammalian subjects, e. g. humans, dogs, cats, and rodents, each unit containing a predetermined quantity of active material calculated to produce the desired pharmaceutical effect in association with the required pharmaceutical diluent, carrier or vehicle. The specifications for the unit dosage forms of this invention are dictated by and dependent on (a) the unique characteristics of the compounds and extracts disclosed above herein and the particular effect to be achieved and (b) the limitations inherent in the art wherein said compounds or extracts are used in humans and animals. Examples of unit dosage forms are tablets, capsules, pills, powder packets, wafers, suppositories, granules, cachets, teaspoonfuls, tablespoonfuls, dropperfuls, ampoules, vials, aerosols with metered discharges, segregated multiples of any of the foregoing, and other forms as herein described. The compositions disclosed herein can be included in kits, which can contain one or more-unit dosage forms of the composition and instructions for use.

Slow or extended-release delivery systems, including any of a number of biopolymers (biological-based systems), systems employing liposomes, colloids, resins, and other polymeric delivery systems or compartmentalized reservoirs, can be utilized with the compositions described herein to provide a continuous or long-term source of the therapeutic compound.

In an embodiment of present invention, the haloperidol, or said pharmaceutically acceptable salt, ester or solvate thereof, and more preferably haloperidol decanoate, or a pharmaceutical composition comprising the same, is administered by transdermal administration route, preferably using a skin patch or by intrathecal administration route, preferably using a pump with intrathecal port catheter. Accordingly, one embodiment of the invention refers to a skin patch comprising haloperidol, or a pharmaceutically acceptable salt, ester or solvate thereof, or a pharmaceutical composition comprising the same, and more preferably comprising a pharmaceutical composition comprising haloperidol and moxifloxacin, or a pharmaceutically acceptable salt, ester or solvate thereof. In particular, one embodiment refers to said skin patch as a medicament and, in particular, for use according to present invention, in the prevention and treatment of spinal muscular atrophy. Said skin patch will slowly release the haloperidol, or the pharmaceutical composition comprising the same, dissolved in an oil with intramuscular delivery (haloperidol decanoate), or even through an intrathecal administration route using a pump with intrathecal port catheter.

## EXAMPLES

The examples of the present invention described below aim to illustrate its preferred embodiments without limiting its scope of protection.

### EXAMPLE 1: Evaluation of haloperidol in SMA fibroblasts

Haloperidol was evaluated in fibroblasts derived from patients with type II spinal muscular atrophy.

### 1.1. Cell culture methods

Fibroblasts derived from SMA patients (GM03813; Coriell Cell Repositories) were grown at 37°C in cell culture bottles with Dulbecco's Modified Eagle Medium (DMEM; Gibco) and 4.5 g/ml glucose. The medium was supplemented with penicillin (100 U/ml) and streptomycin (100 mg/ml) [1% P/S; Gibco] and 10% inactivated fetal bovine serum (FBS; Gibco).

For RNA extraction 400,000 GM03813 fibroblasts were seeded in 60 mm culture dishes in 4 ml of supplemented

DMEM medium. The following day the medium was changed, and the candidate compound was added at its maximum non-toxic concentration. The cells were incubated for 48 hours and then washed with 1x PBS and collected in sterile 1.5 ml tubes. The same was done for the evaluation of the effect of haloperidol on the level of SMN protein (example 2) with the difference that 1,000,000 fibroblasts were used in a 100 mm cell culture plate and that the cells were collected after 2 days with a RIPA protein extraction buffer supplemented with a protease inhibitor cocktail (cOmplete; Roche).

For immunostaining 40,000 fibroblasts cells were seeded in a 400 μl 24-well plate with supplemented DMEM medium followed by medium change with the treatment of the corresponding candidate compound. Cells were incubated for 72 h with candidate compounds and fixed in 4% paraformaldehyde for 10 min. All cell cultures were incubated at 37°C with 5% $CO_2$.

### 1.2. Assays

Fibroblasts derived from SMA type II patients were incubated with haloperidol at three concentrations for 72 h. 10 μg of protein extracted from cells treated with haloperidol were separated by electrophoresis with acrylamide/bis-acrylamide gels SDS-PAGE with the Mini-protean Electrophoresis System (Bio-Rad). After electrophoresis was carried out, the proteins were transferred to a nitrocellulose membrane (GE Healthcare) using a semi-dry electro-transfer system (Bio-Rad) for one hour with a constant voltage of 15V. The membranes were incubated with a blocking solution (5% skim milk in PBST, 1x PBS, 0.1% Tween 20) for at least 1 h at room temperature. They were then incubated at 4°C overnight, with the SMN primary antibody blocking solution, MANSMA1 (11F3; Developmental Studies Hybridoma Bank), and subsequently incubated with a peroxidase-conjugated anti-mouse IgG secondary antibody (Sigma) for 1 hour at room temperatureβ-actin was used as a loading control , using an antibody against β-actin and a peroxidase-conjugated anti-rabbit IgG.

Chemiluminescence was detected using SuperSignal West Pico Sensitivity Substrate (Pierce) and acquired with ImageQuant LAS 4000 (GE Healthcare). The SMN levels were measured by Western blotting and the results obtained were compared to those of a sample treated with solvent (0.5% DMSO). Statistical significance was calculated using the Student's t-test (* p <0.05, ** p <0.01, *** p <0.001).

The results shown in table 2, below, indicate that haloperidol produced a significant increase in SMN protein levels compared to the control with DMSO.

**Table 2:** SMN protein levels

| **DMSO** | **25 μM** | **37.5 μM** | **50 μM** |
|---|---|---|---|
| 0.954352 | 1.131872 | 2.264963 | 2.412854 |
| 0.880324 | 1.750877 | 2.210711 | 2.452798 |
| 1.165324 | 1.745168 | 2.469305 | 2.614414 |
| T-test | 0.0842 (ns) | 0.0003 (***) | 0.0001 (***) |

## EXAMPLE 2: Haloperidol's effect on the SMN2 transcript levels

Haloperidol's effect on the SMN2 transcript was also evaluated.

### 2.1. RNA extraction and reverse transcription.

Extraction of RNA from cells was carried out with ReliaPrep ™ RNA Cell Miniprep System (Promega), following the manufacturer's instructions. This extraction system includes DNase I treatment and, therefore, the reverse transcription of RNA from cells does not include this step. RNA concentration and salt contamination were measured with the NanoDrop spectrophotometer (ThermoFisher). The samples were stored at -80°C. 1 μg of total RNA was retrotranscribed using SuperScript III retrotranscriptase (Invitrogen) following the manufacturer's instructions. Genomic DNA contamination was monitored in the RNA samples without the addition of retrotranscriptase.

### 2.2. PCR reactions (Polymerase Chain Reaction)

For the quantification of the SMN2 FL and Δ7 transcripts, Probeq PCR Mix (Solis Biodyne) was used. The reaction was carried out in a multiplex manner, in which the signal from the SMN2 and GAPDH probe was read in a single reaction. 10 ng of cDNA was used as a standard and the concentration of both probes was 250 nM.

Primer sets for detection of SMN2 FL or Δ7 isoforms were used at a final concentration of 500 nM while the

concentration of the primer set used for GAPDH was 150 nM. All reactions were carried out using a Step-One Real-Time PCR thermocycler (Applied Biosystems). In all cases, the experiment included 3 biological samples and three technical replicates for each sample. Expression levels were normalized to the reference gene using a $2^{-\Delta\Delta Ct}$ method. Primers were obtained from Naryshkin, N. A. et al. Science (2014) 345, 688-693.

**Table 3:** primers set for detection of SMN2 FL or Δ7 isoforms

| | |
|---|---|
| SEQ ID No.: 1 (SMN2 FL Forward) | GCTCACATTCCTTAAATTAAGGAGAAA |
| SEQ ID No.: 2 (SMN2 Δ7 Forward) | TGGCTATCATACTGGCTATTATATGGAA |
| SEQ ID No.: 3 (SMN2 Reverse, both) | TCCAGATCTGTCTGATCGTTTCTT |
| SEQ ID No.: 4 (SMN2 TaqMan probe) | /5' 6-FAM/-CTGGCATAGAGCAGCACTAAATGACACCAC-/3' BHQ_1/ |

**2.3. Assays**

The selected SMA fibroblast line has 3 copies of the SMN2 gene and homozygous deletion of Ex7-Ex8 in SMN1 and therefore only SMN2 gene transcripts were measured. The conditions for cell cultures and extraction protocols are described in Example 1.

Haloperidol increases SMN protein levels and significantly increases the SMN2 FL isoform as shown in table 4 below:

**Table 4.** SMN2 FL and Δ7 isoform levels (results from each of the 3 samples used)

| | **DMSO** | | | **10 μM** | | | **25 μM** | | | **37.5 μM** | | | **50 μM** | | |
|---|---|---|---|---|---|---|---|---|---|---|---|---|---|---|---|
| *SMN2* FL | 0.98 | 0.96 | 1.05 | 1.10 | 1.03 | 1.06 | 1.15 | 1.37 | 1.07 | 1.32 | 1.47 | 1.51 | 2.54 | 2.11 | 2.33 |
| *SMN2* Δ7 | 0.98 | 0.98 | 1.04 | 0.90 | 0.89 | 0.89 | 1.00 | 1.06 | 1.04 | 0.96 | 1.01 | 1.03 | 1.89 | 1.23 | 1.23 |
| T-test | ns | | | ns | | | ns | | | FL=** | | | FL=***/ Δ7=* | | |

As seen in table 4, the FL isoform, which includes exon 7, increases together with haloperidol concentration, while the Δ7 isoform that excludes exon7 does not change in a dose-dependent manner. The quantitative analysis of the isoforms showed that at the highest concentration haloperidol doubled the amount of FL SMN2 transcript which is consistent with what has been observed on the SMN protein level.

**EXAMPLE 3: Evaluation of haloperidol in myoblasts from SMA patients.**

SMN-deficient myoblasts have been reported to have impaired fusion capabilities. We tested haloperidol for its ability to restore myoblasts' ability to form multinuclear myotubes.

**3.1. Cell culture methods**

The myoblasts derived from SMA type II patients (KM1150SMAII7PV; Institut de Myologie, Paris) and the control myoblasts (AB1190; Institut de Myologie, Paris) were grown at 37° C with 5% $CO_2$ in cell culture bottles with medium that consisting of 4 vol of DMEM Glutamax 4.5 g (Gibco) + 1 vol of 199 medium (Gibco) supplemented with 20% inactivated fetal bovine serum (FBS; Gibco), fetuin (25 μg/ml; Life Technologies), hEGF (5 ng/ml ; Life Technologies), bFGF (0.5 ng/ml; Life Technologies), insulin (5 g/ml; Sigma), dexamethasone (0.2 μg/ml; Sigma), penicillin (100 U/ml) and streptomycin (100 mg/ml) [1% P/S; Gibco].

For myoblast immunostaining, 40,000 cells were seeded in a 24-well plate (previously covered with collagen) with 400 μl of KMEM medium and incubated overnight. The following day the medium was changed for the DMEM Glutamax 4.5 g (Gibco) differentiation medium supplemented with 2% horse serum (Sigma); penicillin (100 U/ml) and streptomycin (100 mg/ml) [1% P/S; Gibco], gentamicin (50 μg/ml; Sigma) and insulin (10 μg/ml; Sigma). After 72 hours the medium was changed, and the corresponding compound treatment was administered (2.5 μM haloperidol; 1 μM risdiplam). The cells were incubated for 72 h with the compounds and fixed in 4% paraformaldehyde for 10 minutes. All cell cultures were incubated at 37°C with 5% $CO_2$.

### 3.2. Assays

SMN-deficient myoblasts have been reported to have impaired fusion capabilities. We tested haloperidol for its ability to restore myoblasts' ability to form multinuclear myotubes. For this, we used two immortalized cell lines: SMA type II patient-derived (SMA; ref. KM1150SMAII7PV) and healthy control (CON; ref. AB1190). To do this, myoblasts were treated with 2.5 μM haloperidol for 72 h and the cells were stained for Desmin, a specific protein of muscle cells. Nuclei were stained with DAPI. Myoblasts were seeded in 24-well plates (previously collagen-treated), in 400 μL KMEM, with 40,000 cells per well, and were incubated overnight at 37°C. The following day the cells were changed to differentiation medium in which they were incubated for 3 days. The following day, the cells were changed in medium and treated with DMSO (0.5%), haloperidol at 2.5 μM and risdiplam at 1 μM (positive control) diluted with 0.5% DMSO. The cells were incubated for 72 h and fixed with 4% paraformaldehyde for 10 minutes. The membrane was permeabilized with 1x PBST (1x PBS, 0.3% Triton X-100) for 15 min with subsequent incubation in blocking buffer (1x PBST, 1% normal goat serum) for 1 h at room temperature. Primary monoclonal anti-Desmin antibody (mouse, Millipore) was diluted with blocking buffer (1:50) and incubated overnight at 4°C. The cells were washed 3x with PBS-T (5 min) and biotin-conjugated anti-mouse secondary antibody was diluted with blocking buffer (1: 200) and incubated for 1 h at room temperature. After 3 washes with PBS-T the cells were incubated with ABC solution (ABC kit, VECTASTAIN) for 45 minutes at room temperature, followed by washes with PBS-T and incubation with streptavidin-FITC (1: 200) for 2 h. Finally, the cells were washed with 1x PBS, and the nuclei were stained with DAPI in mounting medium (Vectashield).

It was observed that myoblasts treated with haloperidol significantly increased the fusion index (number of cells with more than 2 nuclei divided by the total number of cells). The fusion index was calculated for healthy cells treated with DMSO 0.5% (CON 0.5%), cells from patients treated with DMSO 0.5%, cells from patients treated with the risdiplam positive control (1 μM) and cells from patients treated with haloperidol (2.5 μM). Healthy myoblasts were observed to have a fusion index of about 68% when patient myoblasts have reduced it to 50%. Both, risdiplam and haloperidol treatment were able to almost fully restore the fusion capabilities of SMA myoblasts, recovering this capacity up to 66.7% (Figure 1A). The images (Figure 1B) were taken with an LSM 800 confocal microscope (Zeiss).

## EXAMPLE 4. Evaluation of haloperidol in motor neurons derived from hiPSCs from SMA patients.

### 4.1. Cell culture methods.

Human induced pluripotent stem cells (hiPSCs) were derived at the Institute for Stem cell Therapy and Exploration of Monogenic diseases (ISTEM) from human primary fibroblasts obtained from Coriell (for the control: GM03814; for SMA: GM00232) and reprogrammed using sendai vectors expressing OCT4, KLF4, SOX2 and c-Myc as described previously [Fusaki et al., 2009].

For spinal motor neurons (MNs) differentiation from hiPSCs, we used a previously reported protocol [Maury et al., 2015]. HiPSC were incubated in suspension in a neural induction medium containing of DMEM/F12 and Neurobasal medium supplemented with B27 (Life Technologies), $N_2$ Supplement (Life Technologies), Pen-Strep 0.1%, β-ME 0.1% (Life Technologies), Ascorbic Acid (0.5 μM, Sigma-Aldrich), Chir99021 (3 μM, Stemgent), SB431542 (20 μM, Tocris), LDN 193189 (0.2 μM, Stemgent) and Y- 27632 (10 μM, Stemcell) at a density of $0.2x10^6$ cells/ml in T25 flask or $0.25x10^6$ cells/ml in spinner flask. After 2 days, caudalization of the neural progenitors was obtained by addition of retinoic acid (RA 0.1 μM) and smoothened agonist (SAG, 0.5 μM). After 1 week in suspension, brain derived neurotrophic factor (BDNF, 10 ng/mL), glial-derived neurotrophic factor (GDNF, 10 ng/mL), and N-[N-(3,5-Difluorophenacetyl)-L-alanyl]-S-phenylglycine t-butyl ester (DAPT, 10 nM) were added in the medium without SB431542 and LDN193189. Between day 10 and 14, MN progenitors are converted into MNs. At this step (day 10) embryoid bodies (EBs) were dissociated into single cells with Trypsin-EDTA and plated at 3.000 cells per well in 384-well plates (Corning) coated with poly-ornithin (20 μg/ml, Sigma-Aldrich) and 5 g/ml laminin (Invitrogen) in 35 μl of the same medium with Y- 27632 (10 μM, Stemcell). Four days later, 35 μl of N2B27 medium containing different concentrations of Haloperidol was added to each well. A solution of 0.1% DMSO (Sigma) was used as a negative control. Cells were treated every 3 days for the next ten days. For each medium change, 32 μl of medium was replaced by 38 μl of N2B27 medium containing the different concentrations of Haloperidol.

### 4.2. Quantification of the effect of Haloperidol on hiPSC-derived MNs survival

Cells plated were fixed using 4% PFA (Electron Microscopy Sciences) for 5 min at room temperature. Fixed cells were incubated with a blocking buffer composed of 1X PBS, 1% BSA (Sigma) and 0.1% Triton-X-100 (Sigma) for 30 min at room temperature. Cells were next incubated overnight at 4°C with primary antibodies (for ISL1: Ref≠ GT15051 Neuromics, 1:1000; TuJI: Ref≠ 801201 Biolegend, 1:1000; SMN: Ref≠ 610647 BDTransduction, 1:200). Each antibody was diluted in a solution of 1X PBS containing 1% BSA. The cells were washed 3 times in PBS and incubated for 1 h

at room temperature with secondary Alexa Fluor- 488, 568, 647-conjugated secondary antibodies diluted to 1:1000 in a solution of 1X PBS containing 1% BSA (ISL1: 568-donkey anti goat Ref≠: A11057, ThermoFisher Scientific; TuJI: 647-donkey anti-rabbit Ref≠: A31573, ThermoFisher Scientific; SMN: 488-donkey anti-mouse Ref≠: A21202 ThermoFisher Scientific). In addition, Hoechst 33228 (1:3000, Sigma) nuclear staining was performed during the secondary labeling. Finally, the cells were washed 3 times in PBS (Figure 2A).

The survival of MNs was determined by quantifying the number of ISL1 positive cells in each condition. ISL1 positive cells were automatically detected using the high-content analysis system Cellomics Array Scan VTI high content imaging system (Thermofisher-Cellomics). Images were acquired with the 20X objective in High Resolution camera mode on two channels: nuclei (channel 1, HOECHST XF93) and ISL1 (channel 2, Texas-red BGRFR_549_15). Acquisition was done for each condition on 4 different wells with 16 fields per well. Based on the Hoechst channel, nuclei were identified using intensity thresholding, background correction and segmentation parameters. Each nuclear region was then reported on the second channel (Texas-red BGRFR_549_15) to limit the ISL1 detection to the nuclear area. ISL1 staining is identified using a fixed intensity threshold, background correction and segmentation parameters.

In this experiment, the MNs were treated for 10 days starting on day 14 from neural induction. A fresh amount of medium with dissolved drugs was delivered every 3 days until day 24 when the MNs were _fixed and immunostained. Healthy MNs were used as a cell survival control as well as a neuroprotective drug kenpaullone, which previously proved to decrease the death rate of SMA MNs in such a designed experimental system.

MN survival was presented as a percentage of ISL1 positive cells counted at day 24 normalized to day 14 of differentiation (Figure 2B). Accordingly, haloperidol administration also resulted in an increased survival rate of motor neurons.

Figure 2A shows representative image of motoneurons stained using the neural marker TUJ1 (class III beta-tubulin) and merge photos for cell nucleus using the fluorescent marker DAPI (4',6-diamidino-2-phenylindole), the MNs differentiation marker ISL1 (Insulin gene enhancer protein) and SMN protein (SMN) after treatment with haloperidol at 320 nM, compared to healthy MNs and DMSO solvent control.

As shown in figure 2B, haloperidol was administered at 8 concentrations, and it proved to almost completely rescue the SMA survival rate at 0.32 μM concentration. Haloperidol activity increasing the SMA motoneurons survival rate was more efficient than the positive control kenpaullone at 10 μM.

SMN protein was also quantified using Homogeneous Time Resolved Fluorescence (HTRF, Cisbio Bioassays) according to the manufacturer's protocol. This technology allows the detection and quantification of SMN protein in 384 well plate format. For this, cells were lyzed in 15 μL of lysate buffer and 10 μL of cell lysate were transferred to a 384-well small volume white plate containing 5 μL of the antibody solution (1:100 dilution of anti-SMN d2 and anti-SMN cryptate) (Cisbio). The plate was incubated overnight at room temperature. Fluorescence was measured at 665 nm and 620 nm on a CLARIOstar microplate reader (BMG Labtech). Ratio values of (665 nm emission/620 nm emission) x 10 000 were used to calculate ΔF (%) according to the following equation:

$$\Delta F\ (\%) = [(\text{Sample ratio} - \text{Negative control ratio}) / \text{Negative control ratio}] \times 100$$

**Example 5. Evaluation of haloperidol on motoneurons and myoblasts co-culture.**

The effect of haloperidol in a co-culture study of SMA type I motoneurons and SMA type I myoblasts was also tested according to the method disclosed in Young et al., 2018 (ISERM, Paris).

Skeletal muscle cells were obtained from a quadriceps muscle biopsy of a 5 day-old unaffected infant (CHQ or Ctl) (Edom et al. 1994), a paravertebral muscle biopsy of a 11-year-old infant patient suffering from SMA (KM432-7PV or SMA type I) and a paravertebral muscle biopsy of a 7-year-old infant patient suffering from SMA (KM1150-11PV or SMA type II). Primary cells were isolated from skeletal muscle as described previously (Chaouch et al. 2009). These cells were expanded and differentiated as previously described. Briefly, primary and immortalized myoblasts were expanded in a mix of 199 medium/Dulbecco modified Eagles minimal essential medium (1:4 ratio) supplemented with 20% fetal bovine serum (FBS), 25 μg/mL fetuin, 0.5 ng/mL basic fibroblast growth factor, 5 ng/mL epidermal growth factor, 5 μg/mL insulin. Cells were then cultivated in a growth medium consisting of DMEM-F12 glutamax (Life Technologies) supplemented with 20% FBS (Sigma-Aldrich) and 0,1% penicillin/steptomycine (Life Technologies). Myogenic differentiation was induced by switching growth medium to DMEM supplemented with 2% horse serum (Life Technologies).

For co-culture, spinal MNs must be differentiated from hiPSC as described previously, but the embryoid bodies are dissociated at day 14 of differentiation and frozen. 384 micropatterned-well plates were designed and provided by CYTOO SA (Young et al. 2018). The myoblasts were plated at a density of 5 500 cells per well in myoblast growth medium (DMEM/F12-glutamax supplemented with 20% fetal bovine serum (Sigma-Aldrich) and 0.1% penicillin-streptomycin (ThermoFisher)) with Y- 27632 (10 μM, Stemcell). After 2 days, the growth medium was replaced by the differentiation medium (DMEM/F12-glutamax supplemented with 2% hors serum (ThermoFisher)). On day 4, the hiPSC-dervied MNs were thawed and plated at a density of 8 000 cells per well in N2B27 medium supplemented by brain derived neurotrophic factor (BDNF, 10 ng/mL, Peprotech), glial-derived neurotrophic factor (GDNF, 10 ng/mL, Peprotech), N-[N-(3,5-Difluorophenacetyl)-L-alanyl]-S-phenylglycine t-butyl ester (DAPT, 10 μM, Tocris) and Y- 27632 (10 μM, Stemcell). Cultures have been maintained up to 11 days which correspond to 4 days of myogenic differentiation and 7 days of co-culture with hiPSC-derived MNs.

After fixation with 4% PFA (Electron Microscopy Sciences) for 7 min at room temperature, cells were incubated overnight at 4°C with primary antibodies (listed in **Table 5**). The cells were washed 3 times in PBS buffer and incubated for 1h at room temperature with appropriated Alexa fluorescent labeled secondary antibodies (Invitrogen, 1:1 000) and Hoechst (1 μg/mL).

**Table 5:** List of primary antibodies, providers and dilutions

| Antibodies | Designation | Reference | Host | Provider | Dilution |
|---|---|---|---|---|---|
| Primary antibodies | Isletl | AF1837 | Goat | R&D systems | 1:500 |
| | Tubulin β III (Tujl) | 801201 | Mouse | Biolegend | 1:1000 |
| | Tubulin β III (Tuj1) | PRB-435P | Rabbit | Biolegend | 1:1000 |
| | Myosin Heavy Chain (MF20) | MF20 | Mouse | DSHB | 1:200 |
| | Acetylcholine receptor (AchR) | mAB 35 | Rat | DSHB | 1:350 |
| | SMN | Sc-32313 | Mouse | Santa Cruz Biotechnology | 1:200 |
| Secondary antibodies | Donkey anti-mouse IgG(H+L) alexa fluor 488 | A21202 | | Thermo Fisher Scientific | 1:1000 |
| | Donkey anti-mouse IgG(H+L) alexa fluor 647 | A-31571 | | Thermo Fisher Scientific | 1:1000 |
| | Donkey anti-goat IgG(H+L) alexa fluor 568 | A-11057 | | Thermo Fisher Scientific | 1:1 000 |
| | Donkey anti-rabbit IgG(H+L) alexa fluor 647 | A-31573 | | Thermo Fisher Scientific | 1:1 000 |
| | Donkey anti-rat IgG(H+L) alexa fluor 488 | A-21208 | | Thermo Fisher Scientific | 1:1000 |
| | Hoechst (1mg/ml) | | | Invitrogen | 1:1 000 |

Co-culture images were acquired using Spinning Disk microscopy (Zeiss) with 20X objective. Each image was taken with z-stack and an orthogonal view was performed on Fiji Software. Islet1 quantification was determined by automatically counting the number of Islet1-positive cells by micropattern. Myotube area was quantified by using Fiji Software to quantify total area of cells stained by MF20 antibody per micropattern. A neuroprotective drug kennpaulone was used as a positive control.

All quantitative data were analyzed with Prism 8. Values are represented as ± SD. Student's t-test was used to compare data between two groups (*$p < 0.05$; **$p < 0.005$; ***$p < 0.0005$; ****$p < 0.0001$). One-way analysis of variance (ANOVA) was performed to compare data among three or more groups, followed by a post-hoc Tukey's multiple comparison test. Values of $p < 0.05$ were considered as statistically significant (*$p < 0.05$; **$p < 0.005$; ***$p < 0.0005$; ****$p < 0.0001$).

Results show that the haloperidol not only increases the survival rate of SMA MNs (figure 3A) but also increases the area of myotubes in respect to not treated controls (figure 3B).

The treatment was administered every 3 days for 7 days from seeding both cell types. It was observed a clear increase in myotubes area (figure 3A) compared with the non-treated SMA cells, which was consistent with the MNs increased survival rate shown in figure 3B.

Figure 3C shows representative images of co-culture treated with haloperidol at 320 nM, compared to solvent-treated wild-type and SMA cells. Myotubes were marked using MF20 marker (dark green), MNs were marked with ISL1 (red), acetylcholine receptors were marked with AChR (light green) and cell nuclei were stained with Hoechst (blue).

**Example 6. Evaluation of haloperidol efficacy in a murine model of SMA (SMN Δ7 mice).**

The efficacy of Haloperidol was then tested (University of Turin, Boido et al.) in SMA Δ7 mice (purchased from Jackson Laboratory, Maine, USA; stock number 005025). This triple mutant mouse harbors two transgenic alleles and a single targeted mutant. The Tg(SMN2*delta7)4299Ahmb allele consists of a SMA cDNA lacking exon 7 whereas the

Tg(SMN2)89Ahmb allele consists of the entire human SMN2 gene. Mice that are homozygous for the targeted mutant Smn allele and homozygous for the two transgenic alleles exhibit symptoms and neuropathology similar to patients afflicted with SMA type II. At birth, triple mutants are noticeably smaller than normal littermates; by day 5, signs of muscle weakness appear and become progressively more pronounced over the following week as the mice display an abnormal gait, shakiness in the hind limbs and a tendency to fall over. Mean survival is approximately 13 days.

To discriminate their genotype, at birth pups were tail snipped and genotyped by PCR.

Treated SMA mice subcutaneously received 0.5 mg/Kg Haloperidol, dissolved in 2% DMSO in saline solution (HALO group), whereas control mice received an equal amount of saline solution with 2% DMSO (VHL group). Haloperidol or saline solution were daily administered, from P2 (postnatal day 2) to P12 (day of sacrifice).

During the whole period, the animals underwent a number of behavioral motor tests: tail suspension test, righting reflex, hindlimb suspension test, negative geotaxis (El-Khodor et al. 2008, Boido et al. 2018). Mice were evaluated daily starting from P2 for body weight and behavioral performances:

- Tail suspension test (self-clasping): pups were suspended by the tail for 15 s, and their hind-limb posture was scored in a scale from 4 (normal) to 0 (clasping);
- Righting reflex: pups were placed on their backs on a flat surface; their success or failure to reposition themselves dorsal side up was assessed over a 30 s period;
- Negative geotaxis test: starting from P4, pups we placed on an inclined grid with the mouse head facing down (cut off: max 60 s).

At P12, Halo and VHL animals were sacrificed, and fresh spinal cords and some skeletal muscles (diaphragm, quadriceps, and gastrocnemius) were rapidly dissected. We then verified the effect of Haloperidol on SMN expression on these samples. Tissues were homogenized on ice using a pestle homogenizer in radioimmunoprecipitation assay buffer (RIPA lysis buffer) (Merck Life Sciences) supplemented with 1 mM PMSF, 1 mM DTT, 2 mM sodium orthovanadate (ThermoFisher Scientific) and 1x cOmpleteTM Protease Inhibitor Cocktail (Merck Life Sciences). Sample homogenates were incubated on ice for 20 min before being centrifuged for the insoluble material separation at 14,000 x g for 20 min at 4 °C. Total protein concentration was assayed on supernatants using Bradford reagent (Bio-Rad). Protein denaturation was performed with NuPAGE® LDS Sample Buffer supplemented with NuPAGE® Sample Reducing Agent (ThermoFisher Scientific), by heating at 95 °C for 5 min. SDS-PAGE and transfer were performed on 4-20% Mini-PROTEAN® TGX™ Precast Protein Gels and Trans-Blot® Turbo™ mini nitrocellulose membranes using a Trans-Blot® Turbo™ transfer System (Bio-Rad), respectively. Nonspecific binding sites were blocked using 5% nonfat dried milk in PBS-0.2% Tween-20 (Merck Life Sciences) (PBS-T) for 1 h at RT under shaking. All membranes were incubated overnight at 4°C under shaking with SMN-antibody solution (mouse-anti-SMN, BD Bioscience 610646) diluted 1:2,000 in 2% nonfat dried milk in PBS-T, and with Vinculin-antibody solution (Monoclonal mouse Anti-Vinculin, Clone Hvin-1, Merck Life Sciences) diluted 1:2,000 in 2% nonfat dried milk in PBS-T as loading control. HRP-conjugated secondary antibody (Goat Anti-Mouse IGG HRP, Bio-Rad) was diluted 1:10,000 in 2% nonfat dried milk in PBS-T and incubated for 1 h at RT under shaking. Immunolabeling was detected with Clarity™ Western ECL Blotting Substrates (Bio-Rad) using the ChemiDocTM imaging system (Bio-Rad). Densitometric quantitation of bands was performed with Fiji (Image J, NIH).

Haloperidol administration supported a significant weight increase in treated SMA mice, starting from P5 (postnatal day 5) and progressively increasing during days (Figure 4A).

Moreover, the behavioral assessment showed that Haloperidol administration in SMA mice improved motor performances in the tail suspension test (Figure 4B), the righting reflex test (Figure 4C) and the negative geotaxis test (Figure 4D): indeed, the treatment significantly ameliorated the hind-limb posture and increased muscle strength and resistance in SMA Halo pups compared to VHL ones.

The behavioral results positively correlated with SMN expression (Figure 5): at P12, spinal cords, diaphragms, quadriceps and gastrocnemius muscles were dissected, and Western Blot was performed to evaluate the protein expression in HALO and VHL groups. Figures 5A, 5B, 5C and 5D show a 58%, 23%, 123% and 102% SMN expression increase in HALO mice compared to VHL mice, respectively, in the spinal cord, diaphragm, quadriceps and gastrocnemius samples.

**Example 7. Combined effect of haloperidol and moxifloxacin on SMA motoneurons survival rate.**

The motoneurons differentiated from the SMA type 1 induced pluripotent stem cells, obtained as disclosed in example 4, were used to evaluate their survival rate after treatment with haloperidol or moxifloxacin. With that purpose, the SMA type 1 MNs were treated with 7 concentrations of each drug separately and the survival rate was measured after 10 days of treatment. We established the most efficient dose (optimal dose) to increase the motoneurons survival when each drug is administered separately (Figure 6).

Next, the MNs were treated with 49 combinations of different concentrations of haloperidol and moxifloxacin

and the survival rate was measured after 10 days of treatment (figure 7). To conduct the assay the same cellular model was used.

The results shown in figure 8 indicate that the combination of haloperidol and moxifloxacin provides an improved activity when administered at lower doses than the optimal doses established for separate administration (obtained in figure 6). Accordingly, the results proved that the combination of both haloperidol and moxifloxacin, could be used at lower doses, while rescuing the motoneurons survival to higher extent than when compared to the drugs administered separately at higher doses (figure 7).

To evaluate the presence of a synergistic effect between haloperidol and moxifloxacin we applied the Bliss Independence model which adopts a probabilistic perspective by assuming that under additivity, the drugs' effects do not interfere with one another. This allows to determine the expected result at a dose combination (d1, d2) as the sum of the independent drug responses minus their joint effect under additivity (in analogy with stochastic independence) (Thas et al., 2021; Van der Borght et al., 2017). The analysis was performed using the R software, specifically:

- R: R Core Team (2021). R: A language and environment for statistical computing. R Foundation for Statistical Computing, Vienna, Austria. URL https://www.R-project.org/.
- BIGL: Heather Turner, Annelies Tourny, Olivier Thas, Maxim Nazarov, Rytis Bagdziunas and Stijn Hawinkel (2021). BIGL: Biochemically Intuitive Generalized Loewe Model. R package version 1.6.4. https://github.com/openanalytics/BIGL

The synergistic and antagonistic effects of drug combinations could be also plotted on a two-dimensional contour plot where the x-axis and y-axis represent doses of haloperidol and moxifloxacin, respectively, as shown in figure 8. Each point follows a grey-scale shown according to the p-value and the sign of the maxR statistic.

Based on the results shown in figure 8, synergy was found when haloperidol and moxifloxacin were administered *in vitro* at doses between 0.04-1.25 μM for haloperidol and between 0.4-12 μM for moxifloxacin.

**REFERENCES**


Boido M, De Amicis E, Valsecchi V, Trevisan M, Ala U, Ruegg MA, Hettwer S, Vercelli A. Increasing Agrin Function Antagonizes Muscle Atrophy and Motor Impairment in Spinal Muscular Atrophy. Front Cell Neurosci. 2018 Jan 30;12:17. doi: 10.3389/fncel.2018.00017.

Chaouch, Soraya, Vincent Mouly, Aurélie Goyenvalle, Adeline Vulin, Kamel Mamchaoui, Elisa Negroni, James Di Santo, et al. 2009. "Immortalized Skin Fibroblasts Expressing Conditional MyoD as a Renewable and Reliable Source of Converted Human Muscle Cells to Assess Therapeutic Strategies for Muscular Dystrophies: Validation of an Exon-Skipping Approach to Restore Dystrophin in Duchenne Muscular Dystrophy Cells." Human Gene Therapy 20 (7): 784-90. https://doi.org/10.1089/hum.2008.163.

Edom, F., V. Mouly, J. P. Barbet, M. Y. Fiszman, and G. S. Butler-Browne. 1994. "Clones of Human Satellite Cells Can Express in Vitro Both Fast and Slow Myosin Heavy Chains." Developmental Biology 164 (1): 219-29. https://doi.org/10.1006/dbio.1994.1193.

El-Khodor BF, Edgar N, Chen A, Winberg ML, Joyce C, Brunner D, Suárez-Fariñas M, Heyes MP. Identification of a battery of tests for drug candidate evaluation in the SMNDelta7 neonate model of spinal muscular atrophy. Exp Neurol. 2008 Jul;212(1):29-43. doi: 10.1016/j.expneurol.2008.02.025.

Fusaki et al., Efficient induction of transgene-free human pluripotent stem cells using a vector based on Sendai virus, an RNA virus that does not integrate into the host genome. Proceedings of the Japan Academy, series B (2009), vol. 85, issue 8, pages 348-362

Livak et al., (2001) "Analysis of relative gene expression data using real-time quantitative PCR and the 2(-Delta Delta C(T)) Method", Methods, vol. 25, issue 4, 402-408.

Maury, Y. et al. Combinatorial analysis of developmental cues efficiently converts human pluripotent stem cells into multiple neuronal subtypes. Nat Biotechnol 33, 89-96 (2015). Naryshkin, N. A., et al. (2014). "Motor neuron disease. SMN2 splicing modifiers improve motor function and longevity in mice with spinal muscular atrophy." Science 345(6197): 688-693. Thas, O., Tourny, A., Verbist, B., Hawinkel, S., Nazarov, M., Mutambanengwe, K., & Bijnens, L. (2021). Statistical detection of synergy: New methods and a comparative study. Pharmaceutical Statistics, pst.2173. https://doi.org/10.1002/pst.2173

Van der Borght, K., Tourny, A., Bagdziunas, R., Thas, O., Nazarov, M., Turner, H., Verbist, B., & Ceulemans, H. (2017). BIGL: Biochemically Intuitive Generalized Loewe null model for prediction of the expected combined effect compatible with partial agonism and antagonism. Scientific Reports, 7(1), 17935. https://doi.org/10.1038/s41598-017-18068-5

Young, Joanne, Yoran Margaron, Mathieu Fernandes, Eve Duchemin-Pelletier, Joris Michaud, Mélanie Flaender, Oana Lorintiu, S6bastien Degot, and Pauline Poydenot. 2018. "MyoScreen, a High-Throughput Phenotypic

Screening Platform Enabling Muscle Drug Discovery." SLAS DISCOVERY: Advancing the Science of Drug Discovery 23 (8): 790-806. https://doi.org/10.1177/2472555218761102

## Claims

1. Haloperidol, or a pharmaceutically acceptable salt, ester or solvate thereof, for use in a method for preventing and/or treating spinal muscular atrophy, wherein said method results in an increase of full length functional SMN protein levels.

2. Haloperidol, or a pharmaceutically acceptable salt, ester or solvate thereof, for use according to claim 1, wherein the spinal muscular atrophy is type I, type II, type III or type IV.

3. Haloperidol, or a pharmaceutically acceptable salt, ester or solvate thereof, for use according to any of claims 1 or 2, wherein said haloperidol, or said pharmaceutically acceptable salt, ester or solvate thereof, is administered prior to, together with, or after administering an additional therapeutic agent for use in the treatment of spinal muscular atrophy.

4. Haloperidol, or a pharmaceutically acceptable salt, ester or solvate thereof, for use according to claim 3, wherein said additional therapeutic agent is selected from the group consisting of: moxifloxacin, nusinersen, onasemnogene abeparvovec, risdiplam, BIIB110, Columbia/NU-p38aMAPK inhibitor, branaplam, monoclonal antibody SRK-015, albuterol, splicing modifier RG7800, recombinant protein RO7239361 and reldesemtiv, or a combination thereof.

5. Haloperidol, or a pharmaceutically acceptable salt, ester or solvate thereof, for use according to any of claims 3 or 4, wherein said additional therapeutic agent is moxifloxacin.

6. Haloperidol, or a pharmaceutically acceptable salt, ester or solvate thereof, for use according to any of claims 3 or 4, wherein said additional therapeutic agent is nusinersen or risdiplam.

7. Haloperidol, or a pharmaceutically acceptable salt, ester or solvate thereof, for use according to any of claims 1 to 6, wherein said pharmaceutically acceptable salt, ester or solvate thereof is haloperidol lactate or haloperidol decanoate.

8. A pharmaceutical composition comprising haloperidol, or a pharmaceutically acceptable salt, ester or solvate thereof, and at least a pharmaceutically acceptable excipient; for use in a method for preventing and/or treating spinal muscular atrophy, wherein said method results in an increase of full length functional SMN protein levels.

9. The pharmaceutical composition for use according to claim 8, wherein said pharmaceutical composition is administered prior to, together with, or after administering an additional therapeutic agent for use in the treatment of spinal muscular atrophy.

10. The pharmaceutical composition for use according to claim 9, wherein said additional therapeutic agent is selected from the group consisting of: moxifloxacin, nusinersen, onasemnogene abeparvovec, risdiplam, BIIB110, Columbia/NU-p38aMAPK inhibitor, branaplam, monoclonal antibody SRK-015, albuterol, splicing modifier RG7800, recombinant protein RO7239361 and reldesemtiv, or a combination thereof.

11. The pharmaceutical composition for use according to any of claims 9 or 10, wherein said additional therapeutic agent is moxifloxacin, or a pharmaceutically acceptable salt, ester or solvate thereof.

12. The pharmaceutical composition for use according to any of claims 8 to 11, wherein said pharmaceutical composition is administered orally, transdermally, intrathecally or parenterally.

13. A pharmaceutical composition comprising haloperidol and moxifloxacin, or a pharmaceutically acceptable salt, ester or solvate thereof, and at least a pharmaceutically acceptable excipient, wherein said pharmaceutical composition comprises a molar ratio of haloperidol:moxifloxacin from 1:5 to 1:10

14. A pharmaceutical composition according to claim 13, for use as a medicament.

**15.** A pharmaceutical composition according to claim 13, for use for use in a method for preventing and/or treating spinal muscular atrophy.

FIGURE 1

A

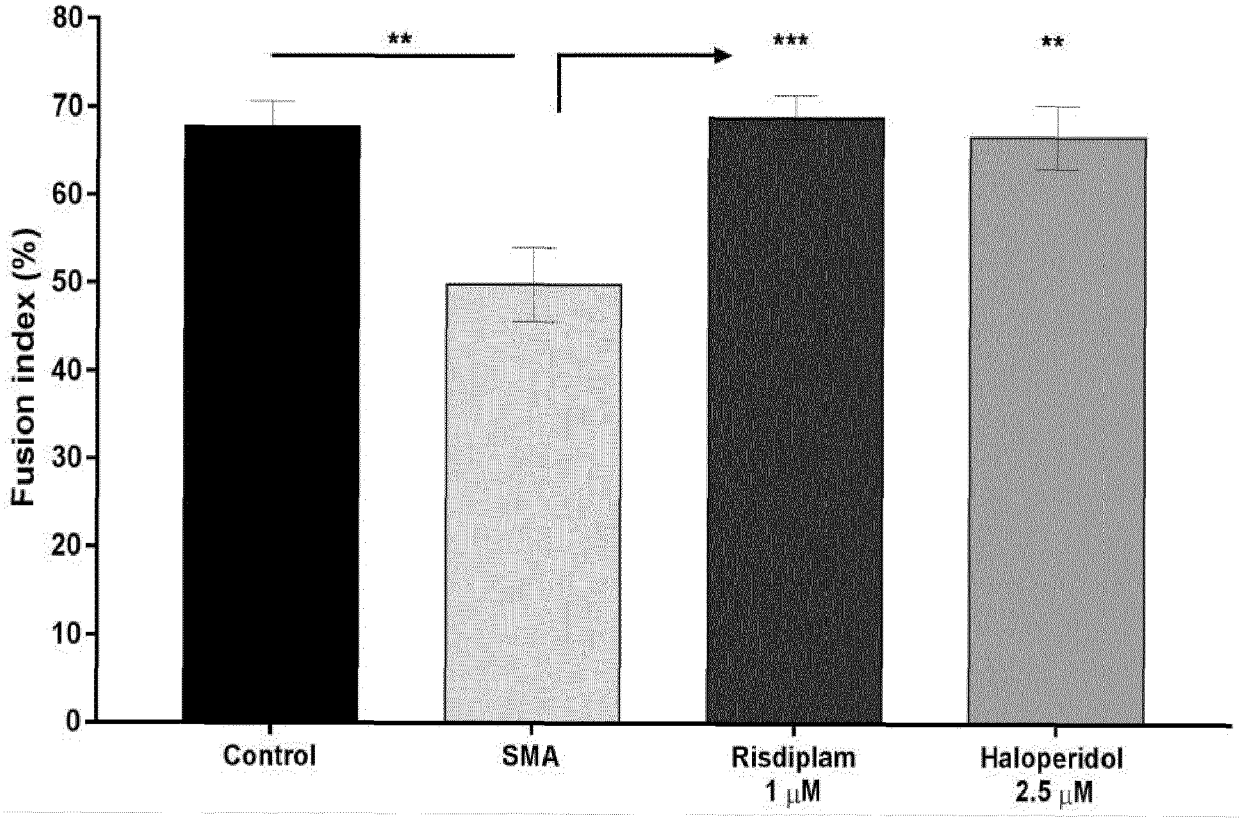
80
70
60
50
40
30
20
10
0
Fusion index (%)
**
***
**
Control
SMA
Risdiplam
1 μM
Haloperidol
2.5 μM

FIGURE 1 (cont.)

B

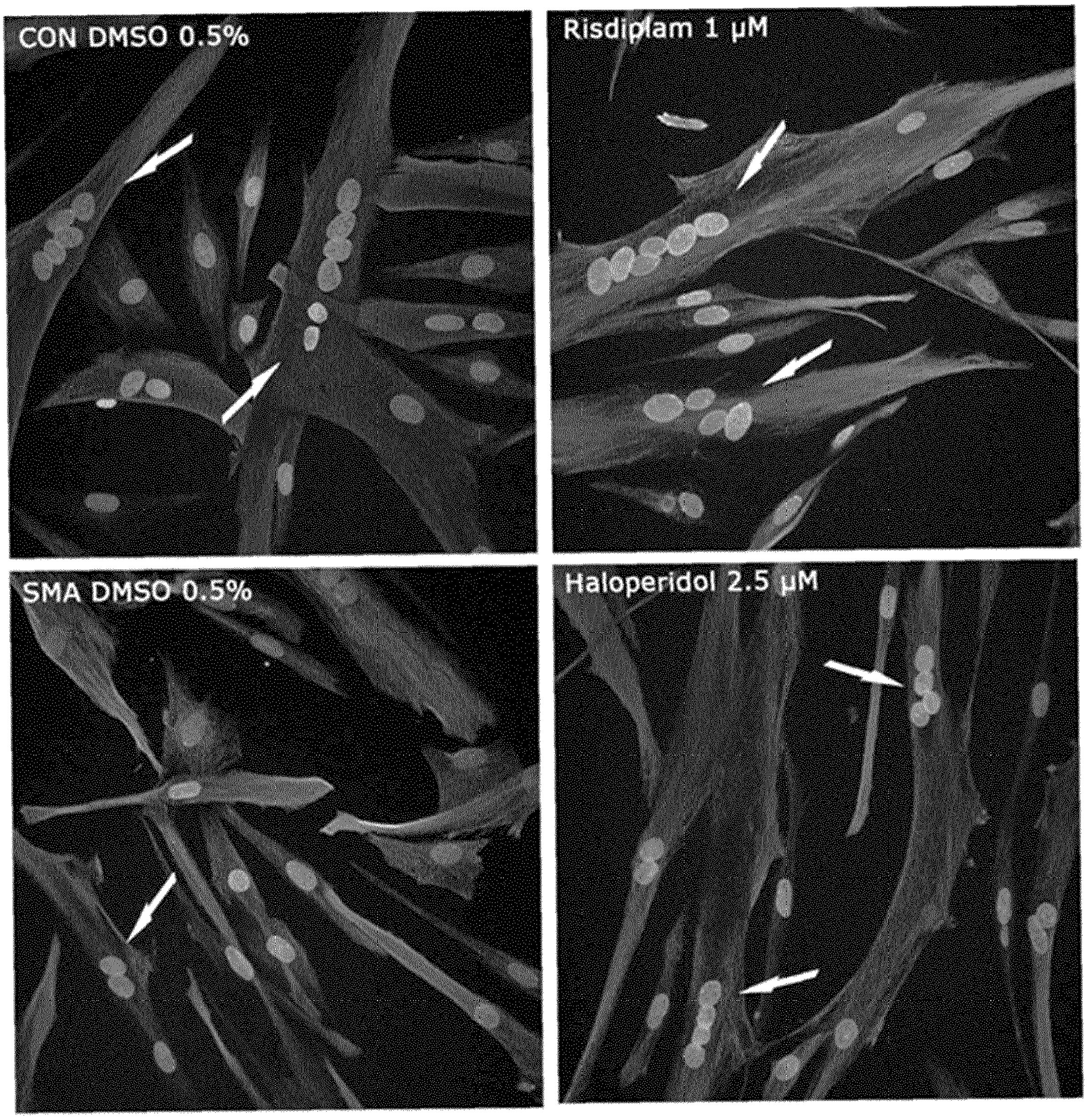

FIGURE 2

A

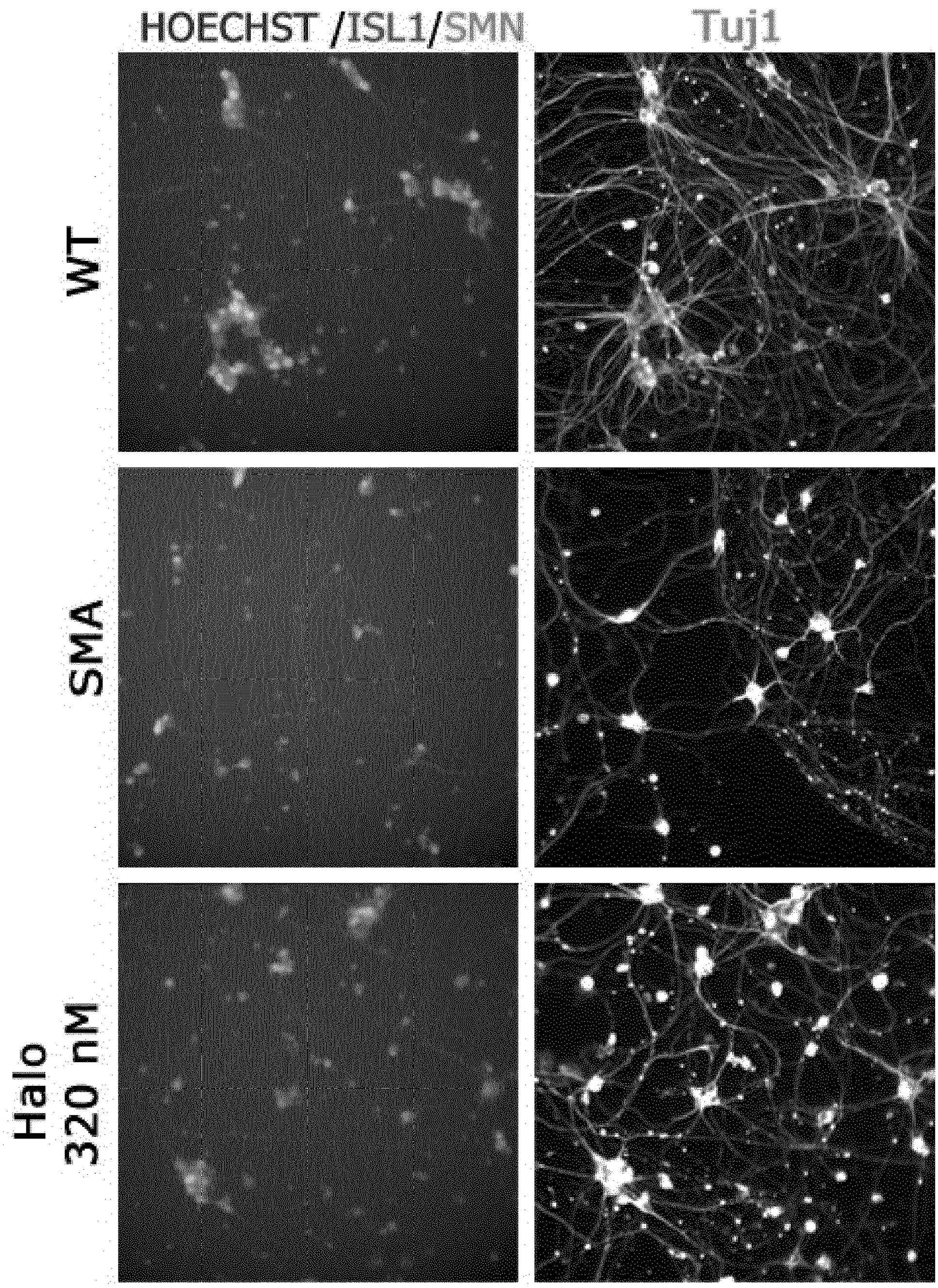

FIGURE 2 (cont.)

B

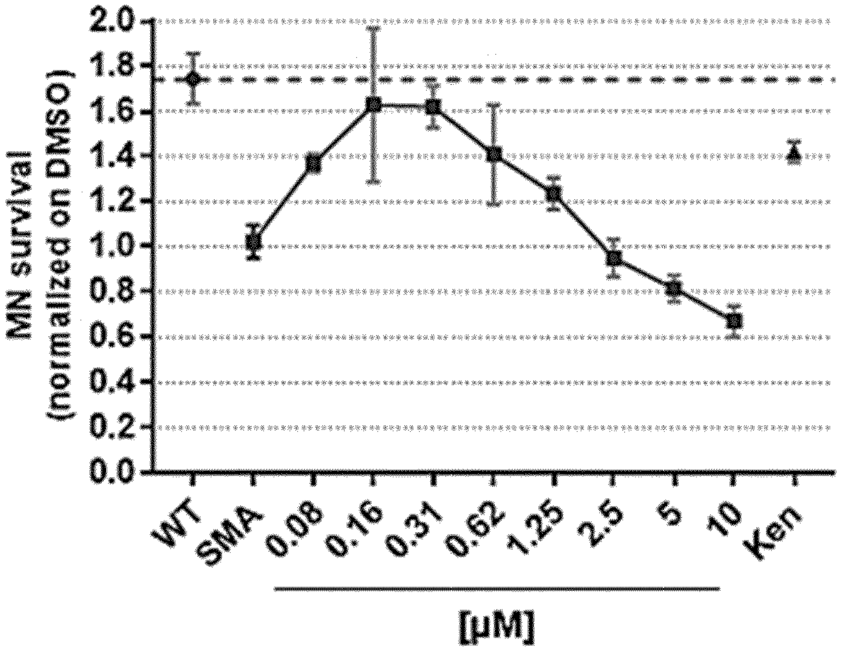


C

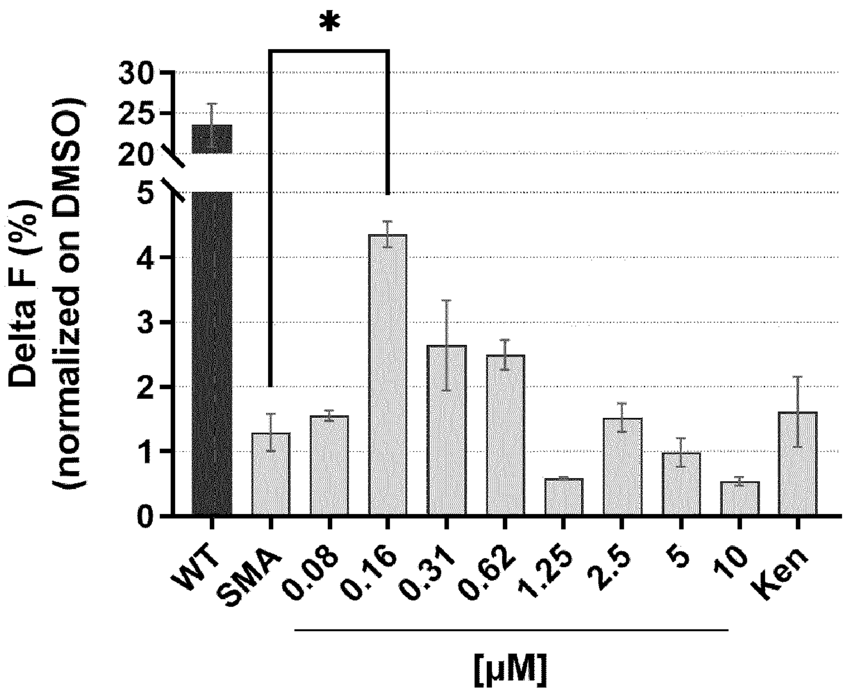

FIGURE 3

A

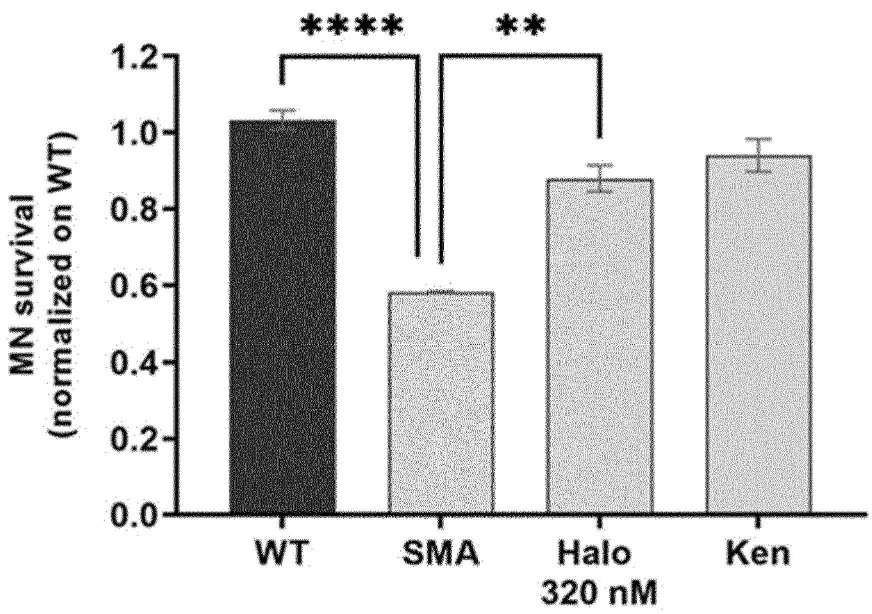

****
**
1.2
1.0
0.8
0.6
0.4
0.2
0.0
MN survival
(normalized on WT)
WT
SMA
Halo
320 nM
Ken


B

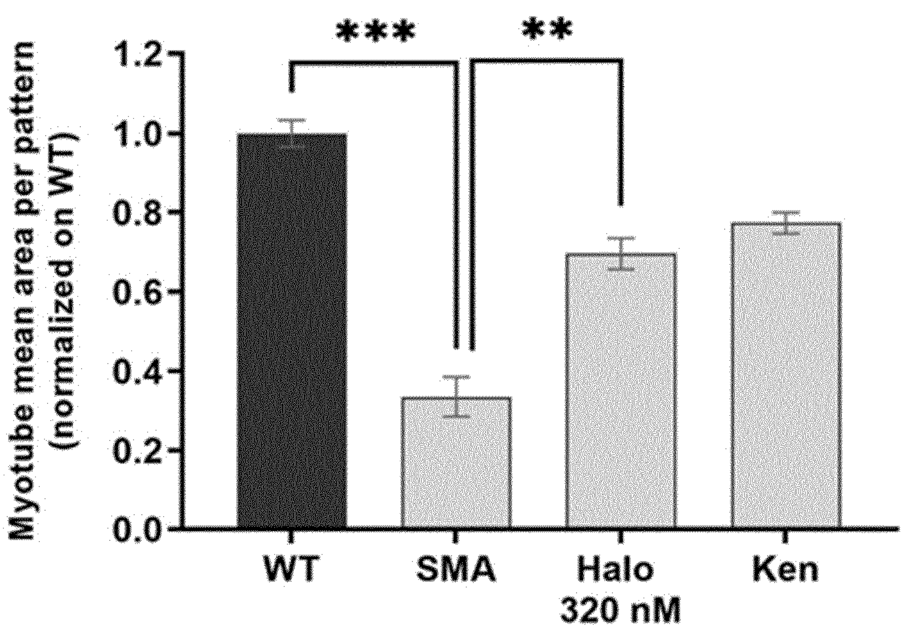

***
**
1.2
1.0
0.8
0.6
0.4
0.2
0.0
Myotube mean area per pattern
(normalized on WT)
WT
SMA
Halo
320 nM
Ken

FIGURE 3 (cont.)

C

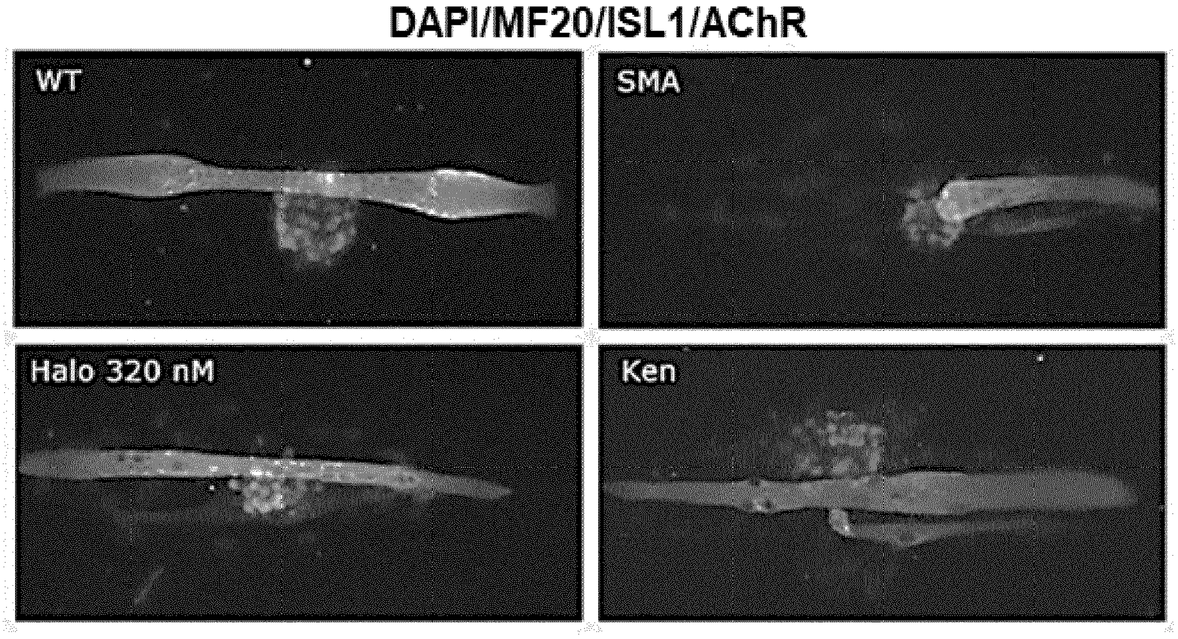


FIGURE 4

A

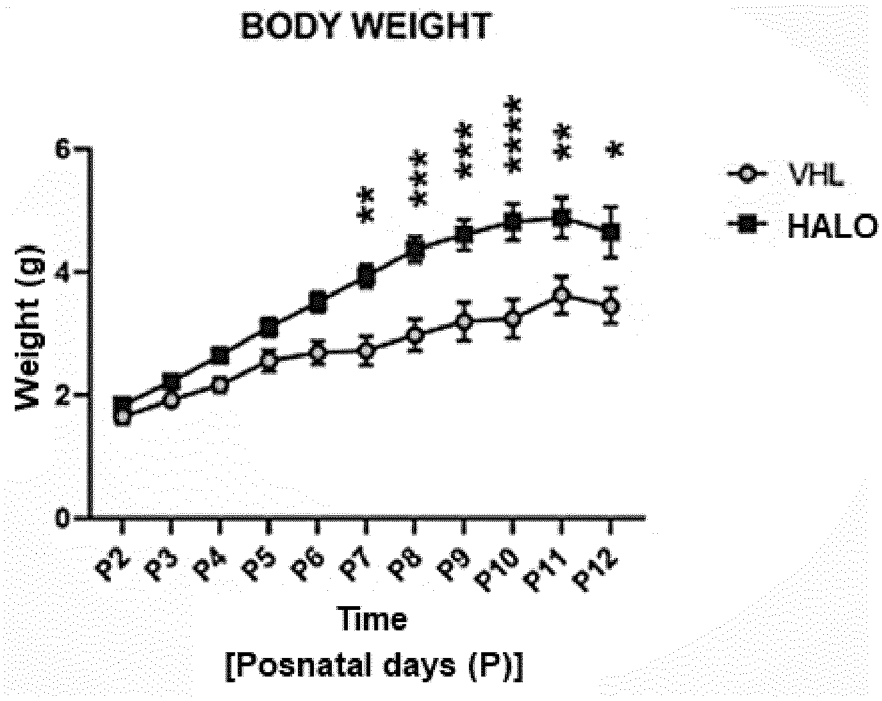

FIGURE 4 (cont.)

B

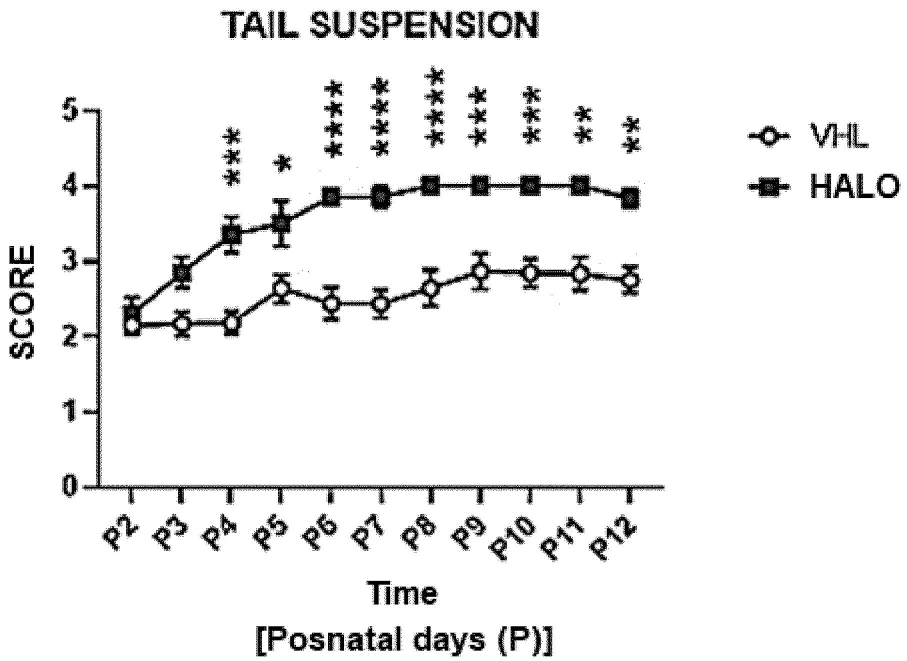


C

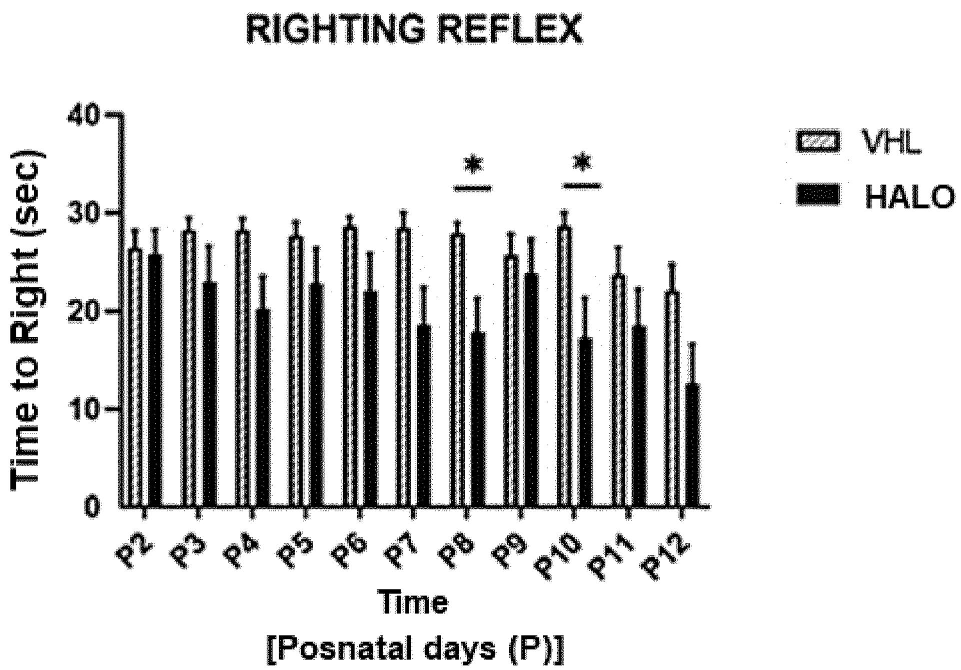

FIGURE 4 (cont.)

D

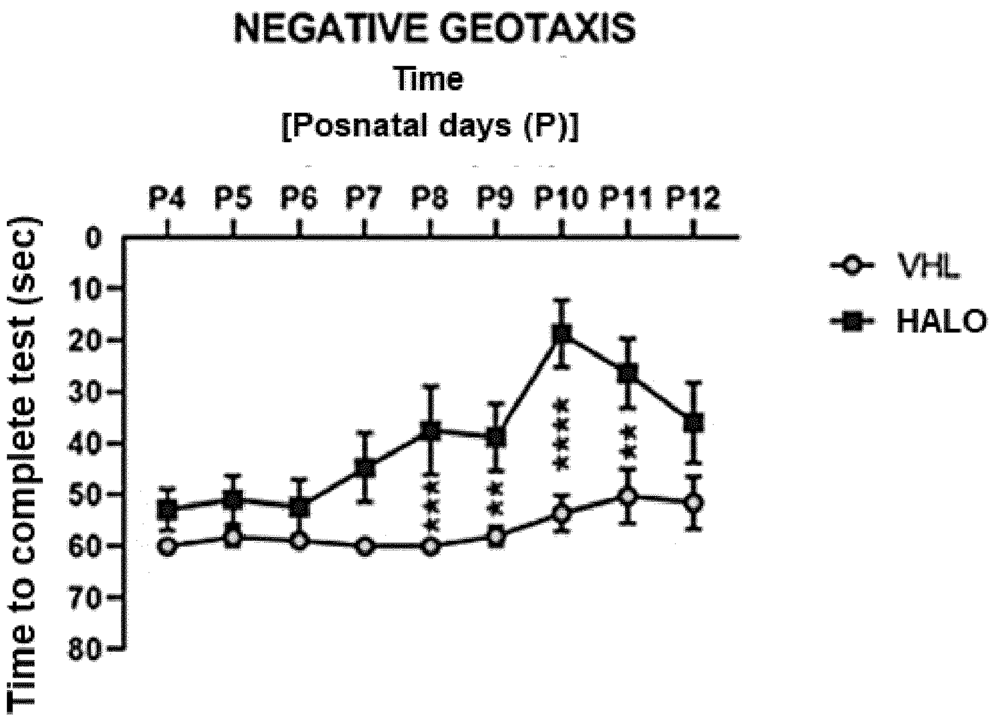

FIGURE 5

A1

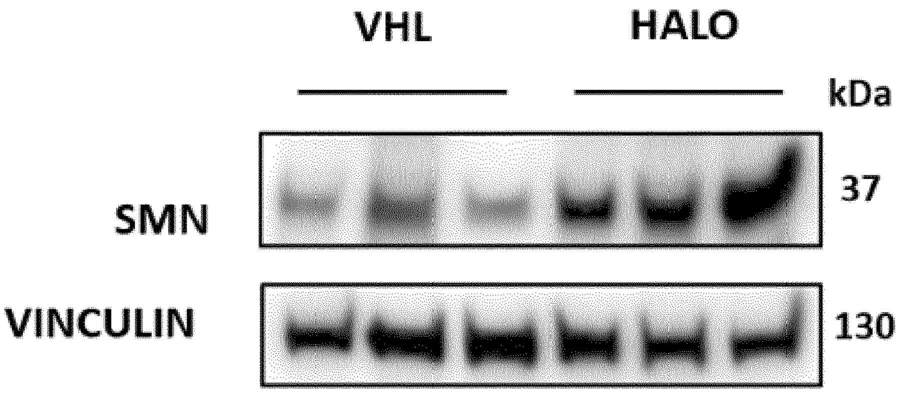


A2

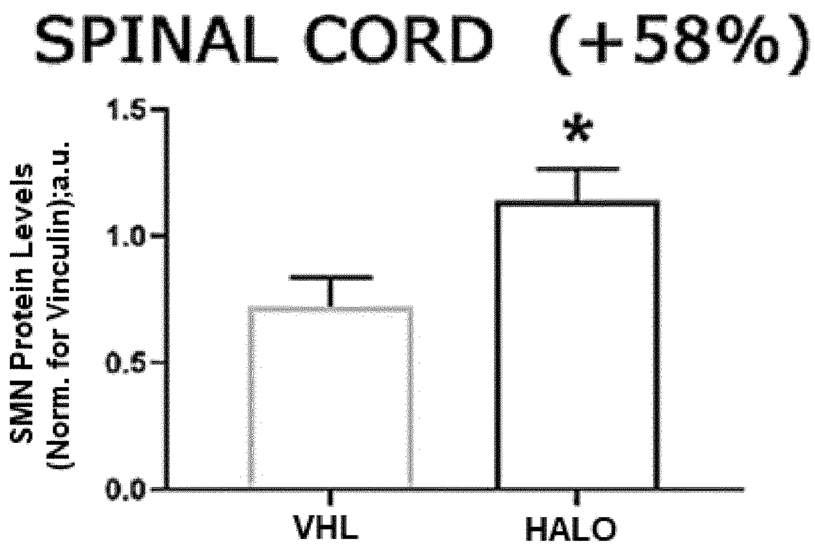

FIGURE 5 (cont.)

B1

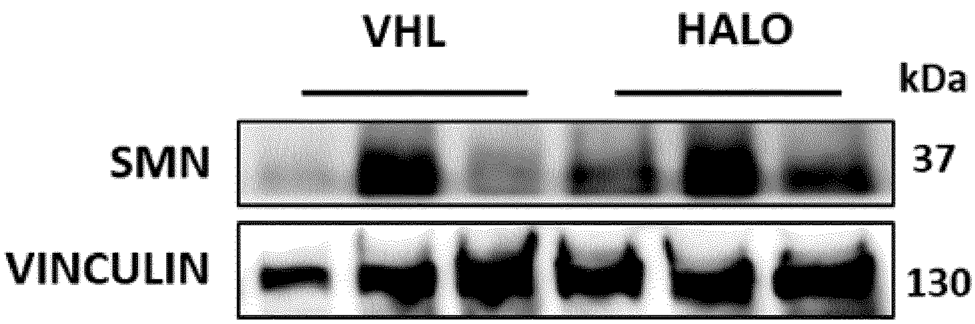


B2

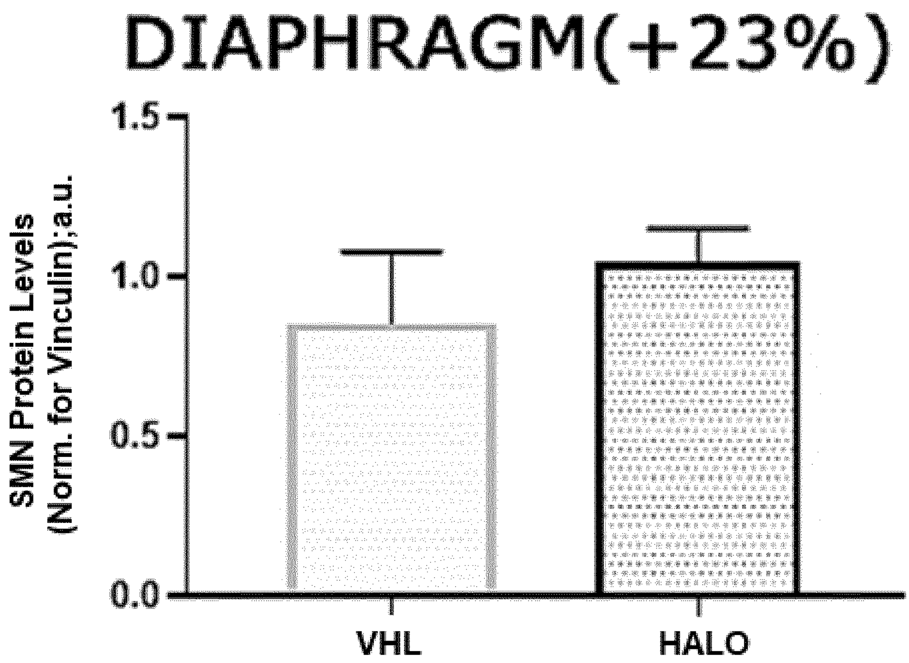

FIGURE 5 (cont.)

C1

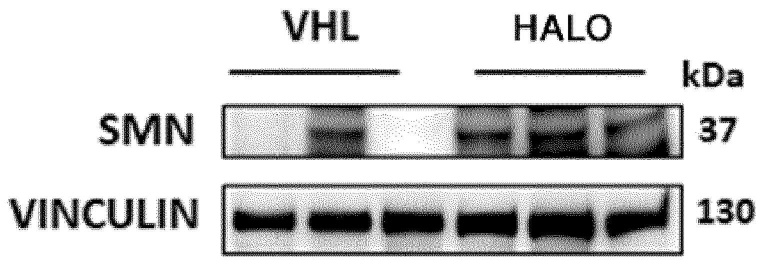


C2

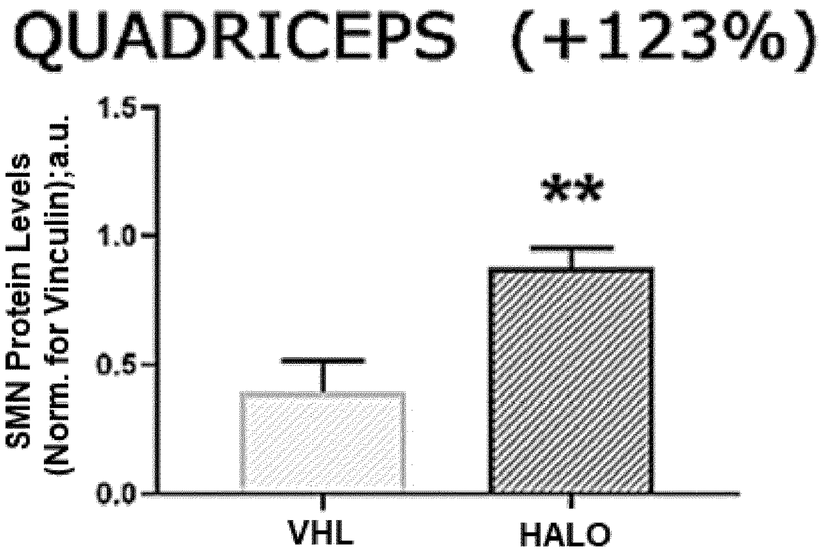

FIGURE 5 (cont.)

D1

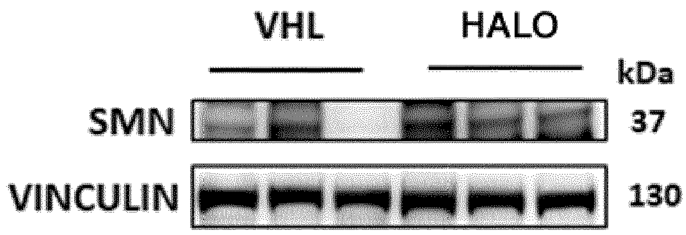


D2

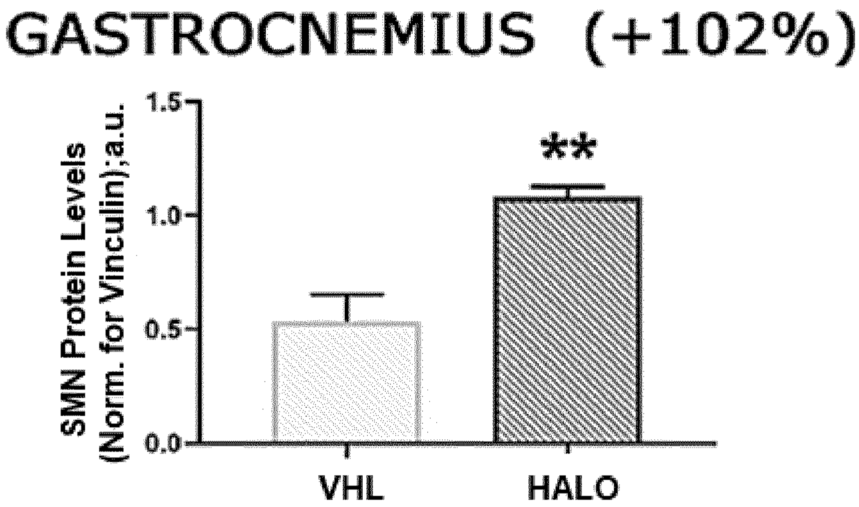


FIGURE 6

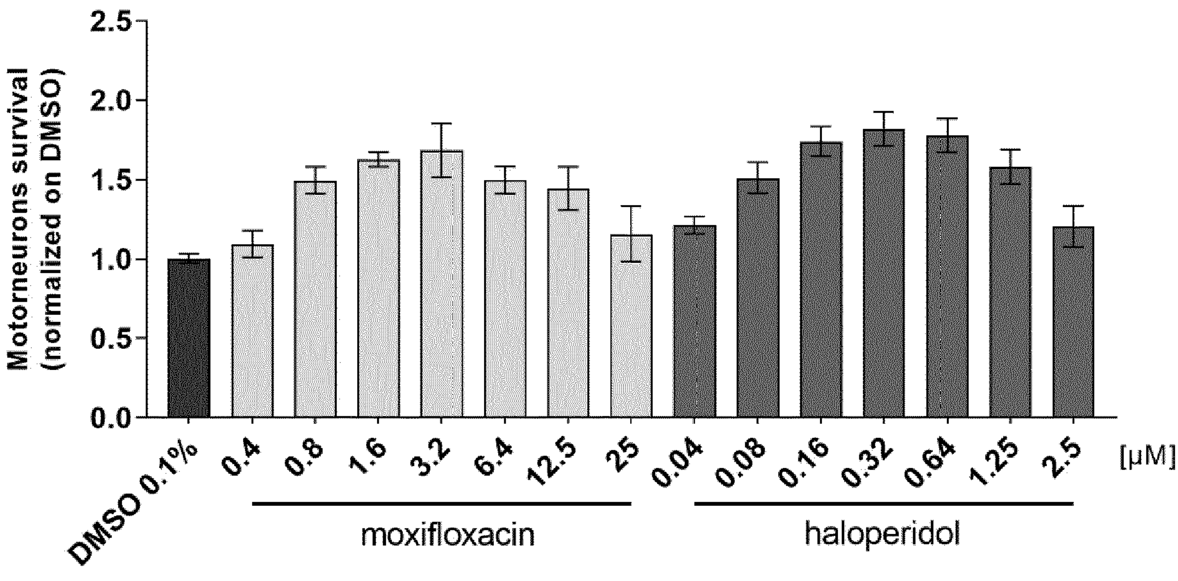

FIGURE 7

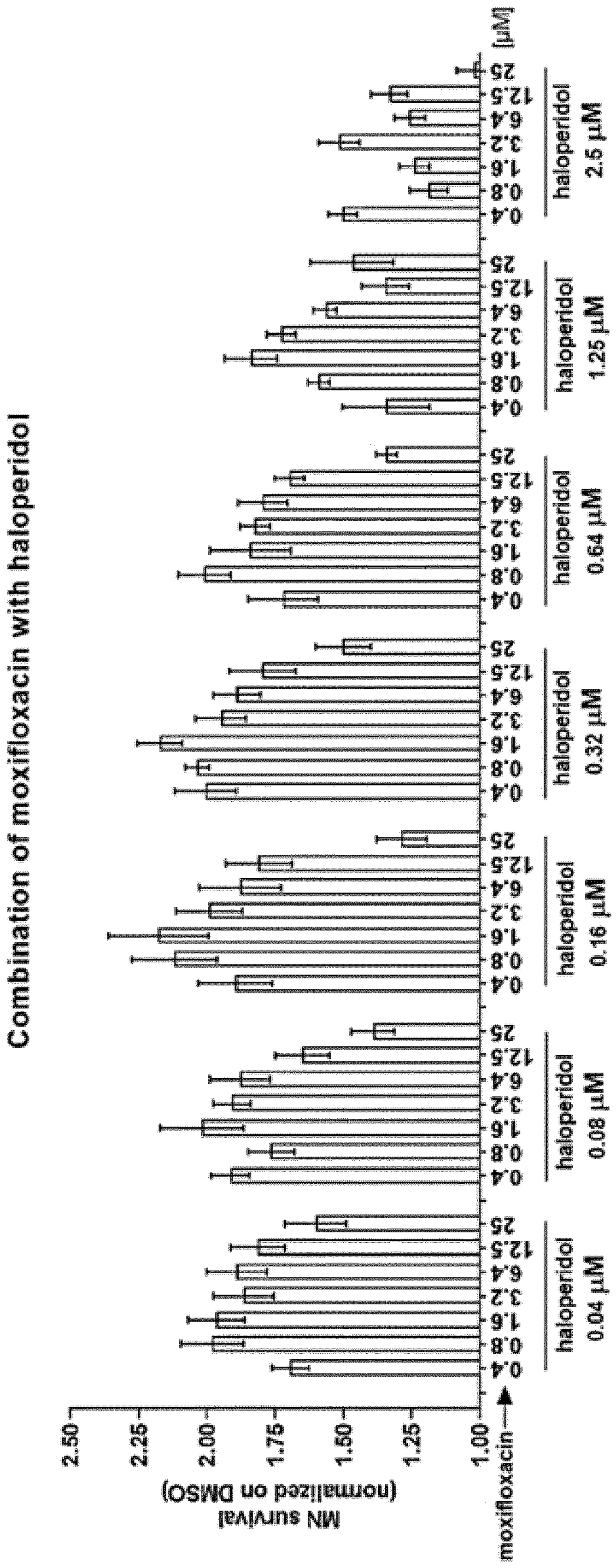
Combination of moxifloxacin with haloperidol
MN survival
(normalized on DMSO)
2.50
2.25
2.00
1.75
1.50
1.25
1.00
moxifloxacin
0.4
0.8
1.6
3.2
6.4
12.5
25
[μM]
haloperidol 0.04 μM
haloperidol 0.08 μM
haloperidol 0.16 μM
haloperidol 0.32 μM
haloperidol 0.64 μM
haloperidol 1.25 μM
haloperidol 2.5 μM

FIGURE 8

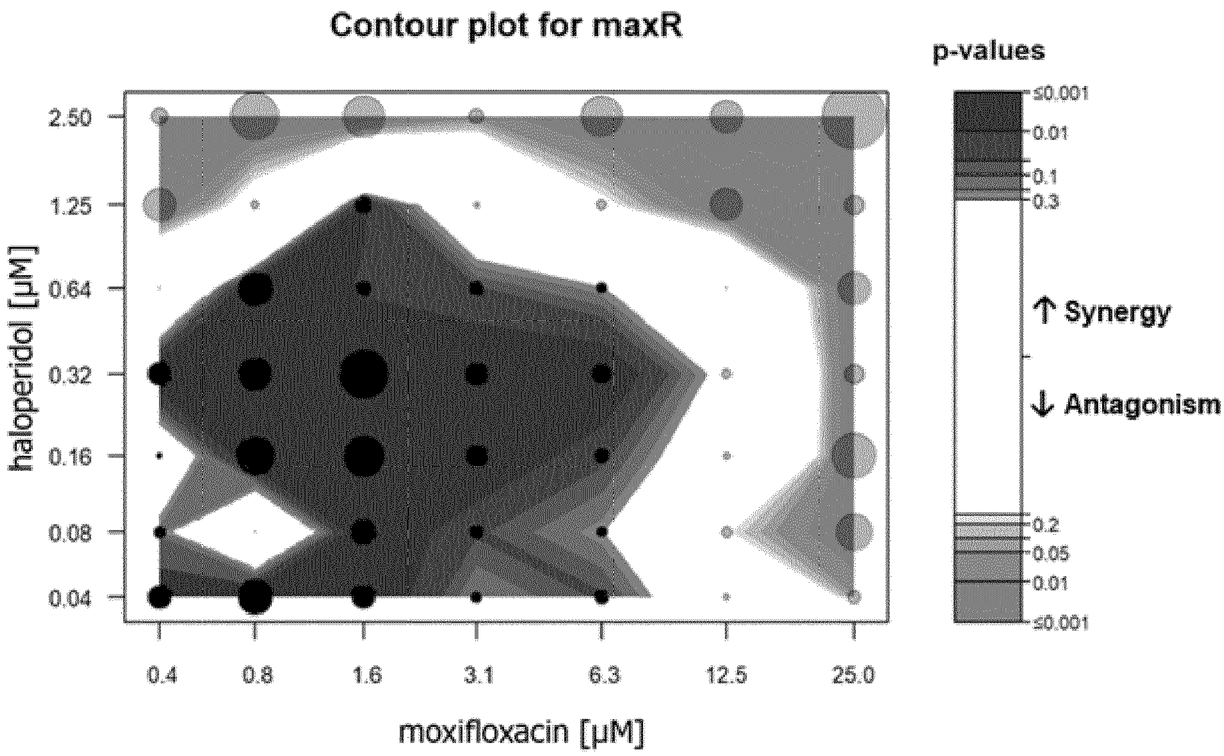
Contour plot for maxR
p-values
2.50
1.25
0.64
0.32
0.16
0.08
0.04
haloperidol [µM]
0.4
0.8
1.6
3.1
6.3
12.5
25.0
moxifloxacin [µM]
≤0.001
0.01
0.1
0.3
↑ Synergy
↓ Antagonism
0.2
0.05
0.01
≤0.001

Europäisches Patentamt
European Patent Office
Office européen des brevets

# EUROPEAN SEARCH REPORT

Application Number
EP 21 38 3162

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MENDUTI GIOVANNA ET AL: "Drug Screening and Drug Repositioning as Promising Therapeutic Approaches for Spinal Muscular Atrophy Treatment", FRONTIERS IN PHARMACOLOGY, vol. 11, 12 November 2020 (2020-11-12), XP055923645, DOI: 10.3389/fphar.2020.592234 Retrieved from the Internet: URL:https://www.ncbi.nlm.nih.gov/pmc/articles/PMC7689316/pdf/fphar-11-592234.pdf> * page 13, right-hand column, lines 3-14 * ----- | 1-15 | INV. A61K31/4515 A61K31/4709 A61K31/519 A61K31/7125 A61P21/00 A61K31/711 |
| A | VITA GIUSEPPE ET AL: "Genetic neuromuscular disorders: living the era of a therapeutic revolution. Part 2: diseases of motor neuron and skeletal muscle", NEUROLOGICAL SCIENCES (TESTO STAMPATO), SPRINGER VERLAG, MILAN, IT, vol. 40, no. 4, 25 February 2019 (2019-02-25), pages 671-681, XP036757919, ISSN: 1590-1874, DOI: 10.1007/S10072-019-03764-Z [retrieved on 2019-02-25] ----- | 1-15 | TECHNICAL FIELDS SEARCHED (IPC): A61K A61P |
| A | RICHARD S. FINKEL ET AL: "Nusinersen versus Sham Control in Infantile-Onset Spinal Muscular Atrophy", THE NEW ENGLAND JOURNAL OF MEDICINE, vol. 377, no. 18, 2 November 2017 (2017-11-02), pages 1723-1732, XP055528610, US ISSN: 0028-4793, DOI: 10.1056/NEJMoa1702752 * results and discussion * ----- -/-- | 1-15 | |

The present search report has been drawn up for all claims

| Place of search | Date of completion of the search | Examiner |
|---|---|---|
| Munich | 24 May 2022 | Venturini, Francesca |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 38 3162

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2020/249577 A1 (HOFFMANN LA ROCHE [CH]; HOFFMANN LA ROCHE [US]) 17 December 2020 (2020-12-17) * claims; examples * ----- | 1-15 | |
| A | US 4 835 152 A (KOROESI JENO [HU] ET AL) 30 May 1989 (1989-05-30) * claims; examples * ----- | 1-15 | |

TECHNICAL FIELDS SEARCHED (IPC)

The present search report has been drawn up for all claims

2

| Place of search | Date of completion of the search | Examiner |
|---|---|---|
| Munich | 24 May 2022 | Venturini, Francesca |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO.

EP 21 38 3162

This annex lists the patent family members relating to the patent documents cited in the above-mentioned European search report. The members are as contained in the European Patent Office EDP file on
The European Patent Office is in no way liable for these particulars which are merely given for the purpose of information.

24-05-2022

| Patent document cited in search report | Publication date | Patent family member(s) | Publication date |
|---|---|---|---|
| WO 2020249577 A1 | 17-12-2020 | CN 113966219 A | 21-01-2022 |
| | | EP 3982970 A1 | 20-04-2022 |
| | | WO 2020249577 A1 | 17-12-2020 |
| US 4835152 A | 30-05-1989 | AU 596206 B2 | 26-04-1990 |
| | | BE 1002415 A4 | 05-02-1991 |
| | | CA 1284322 C | 21-05-1991 |
| | | CH 673652 A5 | 30-03-1990 |
| | | CS 264150 B2 | 13-06-1989 |
| | | DD 265627 A5 | 08-03-1989 |
| | | DE 3727226 A1 | 18-02-1988 |
| | | DK 424887 A | 16-02-1988 |
| | | ES 2004985 A6 | 16-02-1989 |
| | | FI 873525 A | 16-02-1988 |
| | | FR 2602773 A1 | 19-02-1988 |
| | | GB 2194236 A | 02-03-1988 |
| | | HU 198494 B | 30-10-1989 |
| | | IT 1222508 B | 05-09-1990 |
| | | JP S6399073 A | 30-04-1988 |
| | | NL 8701909 A | 01-03-1988 |
| | | NO 164656 B | 23-07-1990 |
| | | PL 148535 B1 | 31-10-1989 |
| | | SE 467357 B | 06-07-1992 |
| | | US 4835152 A | 30-05-1989 |
| | | YU 151487 A | 31-12-1988 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **NARYSHKIN, N. A. et al.** *Science,* 2014, vol. 345, 688-693
- **BOIDO M ; DE AMICIS E ; VALSECCHI V ; TREVISAN M ; ALA U ; RUEGG MA ; HETTWER S ; VERCELLI A.** Increasing Agrin Function Antagonizes Muscle Atrophy and Motor Impairment in Spinal Muscular Atrophy. *Front Cell Neurosci,* 30 January 2018, vol. 12, 17
- **CHAOUCH, SORAYA ; VINCENT MOULY ; AURÉLIE GOYENVALLE ; ADELINE VULIN ; KAMEL MAMCHAOUI ; ELISA NEGRONI ; JAMES DI SANTO et al.** Immortalized Skin Fibroblasts Expressing Conditional MyoD as a Renewable and Reliable Source of Converted Human Muscle Cells to Assess Therapeutic Strategies for Muscular Dystrophies: Validation of an Exon-Skipping Approach to Restore Dystrophin in Duchenne Muscular Dystrophy Cells. *Human Gene Therapy,* 2009, vol. 20 (7), 784-90, https://doi.org/10.1089/hum.2008.163
- **EDOM, F. ; V. MOULY ; J. P. BARBET ; M. Y. FISZMAN ; G. S. BUTLER-BROWNE.** Clones of Human Satellite Cells Can Express in Vitro Both Fast and Slow Myosin Heavy Chains. *Developmental Biology,* 1994, vol. 164 (1), 219-29, https://doi.org/10.1006/dbio.1994.1193
- **EI-KHODOR BF ; EDGAR N ; CHEN A ; WINBERG ML ; JOYCE C ; BRUNNER D ; SUÁREZ-FARIÑAS M ; HEYES MP.** Identification of a battery of tests for drug candidate evaluation in the SMNDelta7 neonate model of spinal muscular atrophy. *Exp Neurol.,* July 2008, vol. 212 (1), 29-43
- **FUSAKI et al.** Efficient induction of transgene-free human pluripotent stem cells using a vector based on Sendai virus, an RNA virus that does not integrate into the host genome. *Proceedings of the Japan Academy,* 2009, vol. 85 (8), 348-362
- **LIVAK et al.** Analysis of relative gene expression data using real-time quantitative PCR and the 2(-Delta Delta C(T)) Method. *Methods,* 2001, vol. 25 (4), 402-408
- **MAURY, Y. et al.** Combinatorial analysis of developmental cues efficiently converts human pluripotent stem cells into multiple neuronal subtypes. *Nat Biotechnol,* 2015, vol. 33, 89-96
- **NARYSHKIN, N. A. et al.** Motor neuron disease. SMN2 splicing modifiers improve motor function and longevity in mice with spinal muscular atrophy. *Science,* 2014, vol. 345 (6197), 688-693
- **THAS, O. ; TOURNY, A. ; VERBIST, B. ; HAWINKEL, S. ; NAZAROV, M. ; MUTAMBANENGWE, K. ; BIJNENS, L.** Statistical detection of synergy: New methods and a comparative study. *Pharmaceutical Statistics,* 2021, https://doi.org/10.1002/pst.2173
- **VAN DER BORGHT, K. ; TOURNY, A. ; BAGDZIUNAS, R. ; THAS, O. ; NAZAROV, M. ; TURNER, H. ; VERBIST, B. ; CEULEMANS, H.** BIGL: Biochemically Intuitive Generalized Loewe null model for prediction of the expected combined effect compatible with partial agonism and antagonism. *Scientific Reports,* 2017, vol. 7 (1), 17935, https://doi.org/10.1038/s41598-017-18068-5
- **YOUNG, JOANNE ; YORAN MARGARON ; MATHIEU FERNANDES ; EVE DUCHEMIN-PELLETIER ; JORIS MICHAUD ; MÉLANIE FLAENDER ; OANA LORINTIU ; S6BASTIEN DEGOT ; PAULINE POYDENOT.** MyoScreen, a High-Throughput Phenotypic Screening Platform Enabling Muscle Drug Discovery. *SLAS DISCOVERY: Advancing the Science of Drug Discovery,* 2018, vol. 23 (8), 790-806, https://doi.org/10.1177/2472555218761102